(19) Europäisches Patentamt
European Patent Office
Office européen des brevets

(11) EP 1 192 131 B1

(12) EUROPEAN PATENT SPECIFICATION

(45) Date of publication and mention of the grant of the patent:
**04.08.2004 Bulletin 2004/32**

(21) Application number: **00938023.9**

(22) Date of filing: **31.05.2000**

(51) Int Cl.[7]: **C07D 209/44**, A61K 31/4035, A61P 43/00, C07D 403/12, C07D 401/12

(86) International application number:
**PCT/US2000/015073**

(87) International publication number:
**WO 2000/076969 (21.12.2000 Gazette 2000/51)**

# (54) METHOD OF INHIBITING AMYLOID PROTEIN AGGREGATION AND IMAGING AMYLOID DEPOSITS USING ISOINDOLINE DERIVATIVES

INHIBITION VON AMYLOID PROTEIN AGGREGATION UND BILDERZEUGUNG VON AMYLOIDNIEDERSCHLÄGEN MIT ISOINDOLDERIVATE

PROCEDE D'INHIBITION D'AGREGATION DE PROTEINES AMYLOIDES ET D'IMAGERIE DE DEPOTS AMYLOIDES AU MOYEN DE DERIVES D'ISOINDOLINE

(84) Designated Contracting States:
**AT BE CH CY DE DK ES FI FR GB GR IE IT LI LU MC NL PT SE**
Designated Extension States:
**AL LT LV MK RO SI**

(30) Priority: **10.06.1999 US 138543 P**

(43) Date of publication of application:
**03.04.2002 Bulletin 2002/14**

(73) Proprietor: **Warner-Lambert Company LLC**
**Morris Plains, New Jersey 07950 (US)**

(72) Inventors:
- **AUGELLI-SZAFRAN, Corinne, Elizabeth**
  **Ann Arbor, MI 48103 (US)**
- **LAI, Yingjie**
  **Edison, NJ 08817 (US)**
- **SAKKAB, Annette, Theresa**
  **Northville, MI 48167 (US)**
- **WALKER, Lary, Craswell**
  **Ann Arbor, MI 48105 (US)**

(74) Representative: **Tesch, Rudolf, Dr. et al**
**Warner-Lambert Company LLC**
**Legal Division, Patent Department,**
**c/o Gödecke GmbH,**
**Mooswaldallee 1**
**79090 Freiburg (DE)**

(56) References cited:
**US-A- 5 552 426**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 192 131 B1

**Description**

FIELD OF THE INVENTION

This invention relates to compounds for inhibiting amyloid protein aggregation and imaging amyloid deposits. More particularly, this invention relates to compounds for inhibiting amyloid protein aggregation in order to treat Alzheimer's disease using isoindoline derivatives.

BACKGROUND OF THE INVENTION

Amyloidosis is a condition characterized by the accumulation of various insoluble, fibrillar proteins in the tissues of a patient. The fibrillar proteins that comprise the accumulations or deposits are called amyloid proteins. While the particular proteins or peptides found in the deposits vary, the presence of fibrillar morphology and a large amount of β-sheet secondary structure is common to many types of amyloids. An amyloid deposit is formed by the aggregation of amyloid proteins, followed by the further combination of aggregates and/or amyloid proteins.

The presence of amyloid deposits has been shown in various diseases, each with its particular associated protein, such as Mediterranean fever, Muckle-Wells syndrome, idiopathetic myeloma, amyloid polyneuropathy, amyloid cardiomyopathy, systemic senile amyloidosis, amyloid polyneuropathy, hereditary cerebral hemorrhage with amyloidosis, Alzheimer's disease, Down's syndrome, Scrapie, Creutzfeldt-Jacob disease, Kuru, Gerstmann-Straussler-Scheinker syndrome, medullary carcinoma of the thyroid, isolated atrial amyloid, $\beta_2$-microglobulin amyloid in dialysis patients, inclusion body myositis, $\beta_2$-amyloid deposits in muscle wasting disease, sickle cell anemia, Parkinson's disease, and Islets ofLangerhans diabetes Type II insulinoma.

Alzheimer's disease is a degenerative brain disorder characterized clinically by progressive loss of memory, cognition, reasoning, judgement, and emotional stability that gradually leads to mental deterioration and ultimately death. Because Alzheimer's disease and related degenerative brain disorders are a major medical issue for an increasingly aging population, the need for new treatments and methods for diagnosing the disorders are needed.

A simple, noninvasive method for detecting and quantitating amyloid deposits in a patient has been eagerly sought. Presently, detection of amyloid deposits involves histological analysis of biopsy or autopsy materials. Both methods have major drawbacks. For example, an autopsy can only be used for a postmortem diagnosis.

The direct imaging of amyloid deposits in vivo is difficult, as the deposits have many of the same physical properties (ie, density and water content) as normal tissues. Attempts to image amyloid deposits directly using magnetic resonance imaging (MRI) and computer-assisted tomography (CAT) have been disappointing and have detected amyloid deposits only under certain favorable conditions. In addition, efforts to label amyloid deposits with antibodies, serum amyloid P protein, or other probe molecules has provided some selectivity on the periphery of tissues, but has provided for poor imaging of tissue interiors.

Thus, it would be useful to have a noninvasive technique for imaging and quantitating amyloid deposits in a patient. In addition, it would be useful to have compounds that inhibit the aggregation of amyloid proteins to form amyloid deposits.

US-A-5 552 426 discloses benzimidazole derivatives for the treatment of Alzeimer's disease.

SUMMARY OF THE INVENTION

The present invention provides compounds having the Formula I:

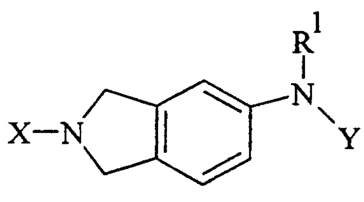

I

or the pharmaceutically acceptable salts thereof,
wherein
X is phenyl or substituted phenyl;
Y is phenyl, substituted phenyl, pyridyl, or substituted pyridyl;
wherein substituted phenyl and substituted pyridyl can have from 1 to 4 substituents, each independently selected from $-OC_1-C_{12}$alkyl, halogen, $-C_1-C_6$alkyl, phenyl,

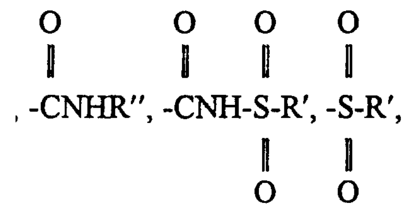

$-CO_2H$, $-CO_2R^1$, $-NO_2$, $-CF_3$, -CN, $-NR^1R^2$, $-(CH_2)_nCO_2H$, $-(CH_2)_nCO_2R^1$, $-SO_2NR^1R^2$, tetrazole, $-(CH_2)_n$-tetrazole, decahydroisoquinoline, imidazole, $-(CH_2)_n$ imidazole, -CH=CH-tetrazole, -CH=CH-imidazole, or phenyl;

$R^1$ and $R^2$ independently are hydrogen or $C_1$-$C_6$alkyl;

each n is independently 0 to 5 inclusive;

R" is hydrogen, $C_1$-$C_6$alkyl, or phenyl; and

R' is hydrogen, $C_1$-$C_6$alkyl, $-CF_3$, or phenyl.

In a preferred embodiment of the compounds of Formula I, X is substituted phenyl and the substituted phenyl has from 1 to 3 substituents independently selected from $-OC_1$-$C_6$alkyl, halogen, $C_1$-$C_6$alkyl, $-CF_3$, or phenyl.

In a preferred embodiment of the compounds of Formula I, Y is substituted phenyl and the substituted phenyl has from to 3 substituents independently selected from $-CO_2H$, $-NO_2$, $-OC_1$-$C_{12}$ alkyl, -CN, tetrazole, $-(CH_2)_nCO_2H$, $-SO_2NR^1R^2$, $-CF_3$, imidazole, $-(CH_2)_n$-tetrazole, $-(CH_2)_n$ imidazole, -CH=CH-tetrazole, or -CH=CH-imidazole.

In a preferred embodiment of the compounds of Formula I, Y is substituted phenyl and the substituted phenyl has from to 3 substituents, one of which is selected from $-CO_2H$.

In a more preferred embodiment of the compounds of Formula I, Y is substituted phenyl, wherein the substituent is $-CO_2H$, which is located at the 2 position of the phenyl ring.

In a more preferred embodiment of the compounds of Formula I wherein X is substituted phenyl, the substituted phenyl has two chlorine substituents located at the 3 and 4 positions of the phenyl ring.

Also provided by the present invention are compounds having the Formula I:

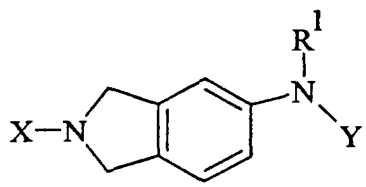

I

or the pharmaceutically acceptable salts thereof,

wherein

X is phenyl or substituted phenyl,

wherein when X is substituted phenyl, the substituted phenyl has from 1 to 4 substituents independently selected from $-OC_1$-$C_6$alkyl, halogen, $C_1$-$C_6$alkyl, $-CF_3$, or phenyl;

Y is phenyl, substituted phenyl, pyridyl, or substituted pyridyl;

wherein when Y is substituted phenyl or substituted pyridyl, the substituted phenyl or substituted pyridyl has from 1 to 4 substituents independently selected from $-CO_2H$, $-NO_2$, $-OC_1$-$C_{12}$alkyl, -CN, $-CF_3$, $-(CH_2)_nCO_2H$, $-SO_2NR^1R^2$, tetrazole, $-(CH_2)_n$-tetrazole, decahydroisoquinoline, phenyl, imidazole, $-(CH_2)_n$ imidazole, -CH=CH-tetrazole, or -CH=CH-imidazole;

$R^1$ and $R^2$ independently are hydrogen or $C_1$-$C_6$alkyl; and

each n is independently 0 to 5 inclusive.

Especially preferred compounds have Formula II

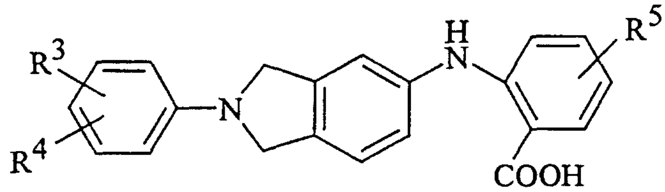

II

and pharmaceutically acceptable salts thereof,
wherein
$R^3$ is halo;
$R^4$ is hydrogen or halo; and
$R^5$ is hydrogen, halo, $C_1$-$C_6$alkyl, -O-$C_1$-$C_6$alkyl, -$CF_3$, -$NO_2$, or-$NR^1R^2$.

Another preferred group of invention compounds have Formula III

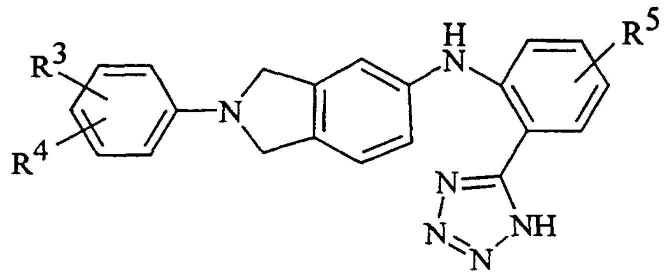

III

and pharmaceutically acceptable salts thereof,
wherein
$R^3$ is halo;
$R^4$ is hydrogen or halo; and
$R^5$ is hydrogen, halo, $C_1$-$C_6$alkyl, -O-$C_1$-$C_6$alkyl, -$CF_3$, -$NO_2$, or-$NR^1R^2$.

Also provided are the compounds:

2-[2-(2,3,4-Trimethoxy-phenyl)-2,3-dihydro-1H-isoindol-5-ylamino]-benzoic acid;
5-Nitro-2-[2-(3,4,5-trimethoxyphenyl)-2,3-dihydro-1H-isoindol-5-ylamino]benzoic acid;
4-Methoxy-5-nitro-2-[2-(3,4,5-trimethoxyphenyl)-2,3-dihydro-1H-isoindol-5-ylamino]benzoic acid;
2-[2-(3,4-Dichlorophenyl)-2,3-dihydro-1H-isoindol-5-ylamino]benzoic acid;
2-[2-(3,4-Dichlorophenyl)-2,3-dihydro-1H-isoindol-5-ylamino]-5-nitrobenzoic acid;
2-[2-(3,4-Dichlorophenyl)-2,3-dihydro-1H-isoindol-5-ylamino]-4-methoxy-5-nitro-benzoic acid;
2-[2-(3-Chlorophenyl)-2,3-dihydro-1H-isoindol-5-ylamino]benzoic acid;
2-[2-(4-Chlorophenyl)-2,3-dihydro-1H-isoindol-5-ylamino]benzoic acid;
2-[2-(3,4-Dimethylphenyl)-2,3-dihydro-1H-isoindol-5-ylamino]benzoic acid;
2-[2-(4-Chloro-3-trifluoromethylphenyl)-2,3-dihydro-1H-isoindol-5-ylamino]benzoic acid;
2-[2-Biphenyl-4-yl-2,3-dihydro-1H-isoindol-5-ylamino]benzoic acid;
2-[2-(3-Chlorophenyl)-2,3-dihydro-1H-isoindol-5-ylamino]-5-nitrobenzoic acid;
2-(2-Phenyl-2,3-dihydro-1H-isoindol-5-ylamino)-benzoic acid;
5-Nitro-2-(2-phenyl-2,3-dihydro-1H-isoindol-5-ylamino)-benzoic acid;
2-[2-(3,4-Dichloro-phenyl)-2,3-dihydro-1H-isoindol-5-ylamino]-benzonitrile;
[2-(3,4-Dichloro-phenyl)-2,3-dihydro-1H-isoindol-5-yl]-(2-tetrazol-1-yl-phenyl)-amine;
{2-[2-(3,4-Dichloro-phenyl)-2,3-dihydro-1H-isoindol-5-ylamino]-phenyl}-acetic acid;
3-{2-[2-(3,4-Dichloro-phenyl)-2,3-dihydro-1H-isoindol-5-ylamino]-phenyl}-propionic acid;
2-[2-(3,4-Dichloro-phenyl)-2,3-dihydro-1H-isoindol-5-ylamino]-6-nitro-benzoic acid;
2-[2-(3,4-Dichloro-phenyl)-2,3-dihydro-1H-isoindol-5-ylamino]-4-nitro-benzoic acid;
2-[2-(3,4-Dichloro-phenyl)-2,3-dihydro-1H-isoindol-5-ylamino]-3-nitro-benzoic acid;
2-[2-(3,4-Dichloro-phenyl)-2,3-dihydro-1H-isoindol-5-ylamino]-5-methanesulfonyl-benzoic acid;
2-[2-(3,4-Dichloro-phenyl)-2,3-dihydro-1H-isoindol-5-ylamino]-5-sulfamoyl-benzoic acid;
4-[2-(3,4-Dichloro-phenyl)-2,3-dihydro-1H-isoindol-5-ylamino]-isophthalic acid;
3-[2-(3,4-Dichloro-phenyl)-2,3-dihydro-1H-isoindol-5-ylamino]-phthalic acid;
2-[2-(3,4-Dichloro-phenyl)-2, 3-dihydro-1H-isoindol-5-ylamino]-5-trifluoromethyl-benzoic acid;
2-[2-(3,4-Dichloro-phenyl)-2,3-dihydro-1H-isoindol-5-ylammo]-5-imidazol-1-yl-benzoic acid;
[2-(3,4-Dichloro-phenyl)-2,3-dihydro-1H-isoindol-5-yl]-(2-tetrazol-1-ylmethyl-phenyl)-amine;
[2-(3,4-Dichloro-phenyl)-2,3-dihydro-1H-isoindol-5-yl]-[2-(2-tetrazol-1-yl-ethyl)-phenyl]-amine;
[2-(3,4-Dichloro-phenyl)-2,3-dihydro-1H-isoindol-5-yl]-[2-(2-tetrazol-1-yl-vinyl)-phenyl)-amine;
2-[2-(3,4-Dichloro-phenyl)-2,3-dihydro-1H-isoindol-5-ylamino]-5-methyl-benzoic acid;
2-[2-(3,4-Dichloro-phenyl)-2,3-dihydro-1H-isoindol-5-ylamino]-3-methyl-benzoic acid;
2-[2-(3,4-Dichloro-phenyl)-2,3-dihydro-1H-isoindol-5-ylamino)-4-nitro-benzoic acid;

2-(2-(3,4-Dichloro-phenyl)-2,3-dihydro-1H-isoindol-5-ylamino]-3,5-dinitro-benzoic acid;
3-[2-(3,4-Dichloro-phenyl)-2,3-dihydro-1H-isoindol-5-ylamino]-2-methyl-benzoic acid;
2-[2-(3,4-Dichloro-phenyl)-2,3-dihydro-1H-isoindol-5-ylamino]-4-methoxy-benzoic acid;
2-[2-(3,4-Dichloro-phenyl)-2,3-dihydro-1H-isoindol-5-ylamino]-4-propoxy-benzoic acid;
4-Butoxy-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino]-4-pentyloxy-benzoic acid;
2-[2-(3,4-Dichloro-phenyl)-2,3-dihydro-1H-isoindol-5-ylamino]-4-hexyloxy-benzoic acid;
2-[2-(3,4-Dichloro-phenyl)-2,3-dihydro-1H-isoindol-5-ylamino]-4-heptyloxy-benzoic acid;
2-[2-(3,4-Dichloro-phenyl)-2,3-dihydro-1H-isoindol-5-ylamino]-4-octyloxy-benzoic acid,
2-[2-(3,4-Dichloro-phenyl)-2,3-dihydro- 1H-isoindol-5-ylamino]-4-nonyloxy-benzoic acid;
4-Decyloxy-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino]-4-isopropoxy-benzoic acid;
2-[2-(4-Chloro-phenyl)-2,3-dihydro-1H-isoindol-5-ylamino]-5-nitro-benzoic acid;
2-[2-(4-Chloro-3-trifluoromethyl-phenyl)-2,3-dihydro-1H-isoindol-5-ylamino]-5-nitro-benzoic acid;
2-(2-Biphenyl-4-yl-2,3-dihydro-1H-isoindol-5-ylamino)-5-nitro-benzoic acid; or
2-[2-(3,4-Dimethyl-phenyl)-2,3-dihydro-1H-isoindol-5-ylamino]-5-nitro-benzoic acid.

Also provided is a pharmaceutical composition comprising a compound of Formula I together with a pharmaceutically acceptable carrier, diluent, or excipient therefor.
Also provided are compounds for the manufacturing of pharmaceuticals for treating Alzheimer's disease.
Also provided are compounds for the manufacturing of pharmaceuticals for inhibiting the aggregation of amyloid proteins to form amyloid deposits.
Also provided are labeled compounds for imaging amyloid deposits.
In a preferred embodiment the disease is Alzheimer's disease.
In a preferred embodiment the labeled compound is a radiolabeled compound.
In a preferred embodiment the labeled compound is detected using MRI.

DETAILED DESCRIPTION OF THE INVENTION

The term "alkyl" means a straight or branched chain hydrocarbon. Representative examples of alkyl groups are methyl, ethyl, propyl, isopropyl, isobutyl, butyl, tert-butyl, sec-butyl, pentyl, and hexyl.
Preferred alkyl groups are $C_1$-$C_6$ alkyl.
The term "alkoxy" means an alkyl group attached to an oxygen atom. Representative examples of alkoxy groups include methoxy, ethoxy, tert-butoxy, propoxy, and isobutoxy.
The term "halogen" includes chlorine, fluorine, bromine, and iodine.
The term "substituted" means that one or more hydrogen atom in a molecule has been replaced with another atom or group of atoms. For example, substituents include halogen, -OH, -$CF_3$, -$NO_2$, -$NH_2$, -NH($C_1$-$C_6$alkyl), -N($C_1$-$C_6$alkyl)$_2$, $C_1$-$C_6$ alkyl, -O$C_1$-$C_6$ alkyl, -CN, -$CF_3$, -$CO_2$H, and -$CO_2C_1$-$C_6$ alkyl.
The term "substituted phenyl" means a phenyl ring in which from 1 to 4 hydrogen atoms have been independently replaced with a substituent, preferably one selected from the list above. Examples include 3-chlorophenyl, 2,6-dibromophenyl, 4-nitrophenyl, 3,4,5-trimethoxyphenyl, and 3-diethylaminophenyl.
The symbol "-" means a covalent bond
The term "pharmaceutically acceptable salt, ester, amide, and prodrug" as used herein refers to those carboxylate salts, amino acid addition salts, esters, amides, and prodrugs of the compounds of the present invention which are, within the scope of sound medical judgement, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively nontoxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate and laurylsulphonate salts, and the like. These may include cations based on the alkali and alkaline earth metals, such as sodium, lithium, potassium, calcium, magnesium, and the like, as well as, nontoxic ammonium, quaternary ammonium and amine cations including, but not limited to ammonium, tetramethylammonium, tetraethylammonium, methylamine, dimethylamine, trimethylamine, triethylamine, ethylamine, and the like. (See, for example, Berge S.M., et al., Pharmaceutical Salts, *J. Pharm. Sci.,* 66:1-19 (1977) which is incorporated herein by reference.)

Examples of pharmaceutically acceptable, nontoxic esters of the compounds of this invention include $C_1$-$C_6$ alkyl esters wherein the alkyl group is a straight or branched chain. Acceptable esters also include $C_5$-$C_7$ cycloalkyl esters as well as arylalkyl esters such as, but not limited to benzyl. $C_1$-$C_4$ alkyl esters are preferred. Esters of the compounds of the present invention may be prepared according to conventional methods.

Examples of pharmaceutically acceptable, nontoxic amides of the compounds of this invention include amides derived from ammonia, primary $C_1$-$C_6$ alkyl amines and secondary $C_1$-$C_6$ dialkyl amines wherein the alkyl groups are straight or branched chain. In the case of secondary amines, the amine may also be in the form of a 5- or 6-membered heterocycle containing one nitrogen atom. Amides derived from ammonia, $C_1$-$C_3$ alkyl primary amides and $C_1$-$C_2$ dialkyl secondary amides are preferred. Amides of the compounds of the invention may be prepared according to conventional methods.

The term "prodrug" refers to compounds that are rapidly transformed in vivo to yield the parent compound of the above formulas, for example, by hydrolysis in blood. A thorough discussion is provided in T. Higuchi and V. Stella, Pro-drugs as Novel Delivery Systems, Vol. 14 of the A.C.S. Symposium Series, and in Bioreversible Carriers in Drug Design, ed. Edward B. Roche, American Pharmaceutical Association and Pergamon Press, 1987, both of which are incorporated herein by reference.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

The compounds of the present invention can exist in different stereoisometric forms by virtue of the presence of asymmetric centers in the compounds. It is contemplated that all stereoisometric forms of the compounds, as well as mixture thereof, including racemic mixtures, form part of this invention.

In the first step of the present method of imaging, a labeled compound of Formula I is introduced into a tissue or a patient in a detectable quantity. The compound is typically part of a pharmaceutical composition and is administered to the tissue or the patient by methods well-known to those skilled in the art.

In the methods of the present invention, a compound can be administered either orally, rectally, parenterally (intravenous, by intramuscularly or subcutaneously), intracisternally, intravaginally, intraperitoneally, intravesically, locally (powders, ointments or drops), or as a buccal or nasal spray.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles include water, ethanol, polyols (propyleneglycol, polyethyleneglycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft- and hard-filled gelatin capsules using such excipients as lactose or milk sugar, as well as high molecular weight polyethyleneglycols, and the like.

Solid dosage forms such as tablets, dragees, capsules, pills, and granules can be prepared with coatings and shells, such as enteric coatings and others well known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, and sesame oil, glycerol, tetrahydrofurfuryl alcohol, polyethyleneglycols, and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compounds of the present invention with suitable nonirritating excipients or carriers such as cocoa butter, polyethyleneglycol or a suppository wax, which are solid at ordinary temperatures but liquid at body temperature and therefore, melt in the rectum or vaginal cavity and release the active component.

Dosage forms for topical administration of a compound of this invention include ointments, powders, sprays, and inhalants. The active component is admixed under sterile conditions with a physiologically acceptable carrier and any preservatives, buffers or propellants as may be required. Ophthalmic formulations, eye ointments, powders, and solutions are also contemplated as being within the scope of this invention.

The labeled compound is introduced into a patient in a detectable quantity and after sufficient time has passed for the compound to become associated with amyloid deposits, the labeled compound is detected noninvasively inside the patient. A labeled compound of Formula I is introduced into a patient, sufficient time is allowed for the compound to become associated with amyloid deposits, and then a sample of tissue from the patient is removed and the labeled compound in the tissue is detected apart from the patient. A tissue sample is removed from a patient and a labeled compound of Formula I is introduced into the tissue sample. After a sufficient amount of time for the compound to become bound to amyloid deposits, the compound is detected.

The administration of the labeled compound to a patient can be by a general or local administration route. For example, the labeled compound may be administered to the patient such that it is delivered throughout the body. Alternatively, the labeled compound can be administered to a specific organ or tissue of interest. For example, it is desirable to locate and quantitate amyloid deposits in the brain in order to diagnose or track the progress of Alzheimer's disease in a patient.

The term "tissue" means a part of a patient's body. Examples of tissues include the brain, heart, liver, blood vessels, and arteries. A detectable quantity is a quantity of labeled compound necessary to be detected by the detection method chosen. The amount of a labeled compound to be introduced into a patient in order to provide for detection can readily be determined by those skilled in the art. For example, increasing amounts of the labeled compound can be given to a patient until the compound is detected by the detection method of choice. A label is introduced into the compounds to provide for detection of the compounds.

The term "patient" means humans and other animals. Those skilled in the art are also familiar with determining the amount of time sufficient for a compound to become associated with amyloid deposits. The amount of time necessary can easily be determined by introducing a detectable amount of a labeled compound of Formula I into a patient and then detecting the labeled compound at various times after administration. A labeled compound of Formula I is any compound thereof having at least one radioactive element as part of the structure.

The term "associated" means a chemical interaction between the labeled compound and the amyloid deposit. Examples of associations include covalent bonds, ionic bonds, hydrophilic-hydrophilic interactions, hydrophobic-hydrophobic interactions, and complexes.

Those skilled in the art are familiar with the various ways to detect labeled compounds. For example, magnetic resonance imaging (MRI), positron emission tomography (PET), or single photon emission computed tomography (SPECT) can be used to detect radiolabeled compounds. The label that is introduced into the compound will depend on the detection method desired. For example, if PET is selected as a detection method, the compound must possess a positron-emitting atom, such as $^{11}$ C or $^{18}$F.

Another example of a suitable label in a compound of Formula I is an atom such as $^{13}$C, $^{15}$N, or $^{19}$F which can be detected using magnetic resonance imaging (MRI) which is also sometimes called nuclear magnetic resonance (NMR). In addition, the labeled compounds of Formula I may also be detected by MRI using paramagnetic contrast agents. Compounds having a radioactive element as part of its structure are readily prepared by standard synthetic methods.

Another example of detection is electron paramagnetic resonance (EPR). In this case, EPR probes which are well-known in the art, such as nitroxides, can be used.

The imaging of amyloid deposits can also be carried out quantitatively so that the amount of amyloid deposits can be determined.

The present invention also provides compounds for inhibiting the aggregation of amyloid proteins to form amyloid deposits, which can be administered to a patient in need of inhibition of the aggregation of amyloid protein. Those skilled in the art are readily able to determine an amyloid inhibiting amount by simply administering a compound of Formula I to a patient in increasing amounts until the growth of amyloid deposits is decreased or stopped. The rate of growth can be assessed using imaging or by taking a tissue sample from a patient and observing the amyloid deposits therein.

A patient in need of inhibition of the aggregation of amyloid proteins is a patient having a disease or condition in which amyloid proteins aggregate. Examples of such diseases and conditions include Mediterranean fever, Muckle-Wells syndrome, idiopathetic myeloma, amyloid polyneuropathy, amyloid cardiomyopathy, systemic senile amyloidosis, amyloid polyneuropathy, hereditary cerebral hemorrhage with amyloidosis, Alzheimer's disease, Down's syndrome, Scrapie, Creutzfeldt-Jacob disease, Kuru, Gerstmann-Straussler-Scheinker syndrome, medullary carcinoma of the thyroid, isolated atrial amyloid, $\beta_2$-microglobulin amyloid in dialysis patients, inclusion body myositis, $\beta_2$-amyloid deposits in muscle wasting disease, sickle cell anemia, Parkinson's disease, and Islets of Langerhans diabetes type 2 insulinoma.

Also provided by the present invention are labeled compounds of Formula I, wherein one or more atoms in the compound has been replaced with a radioisotope. The radioisotope can be any radioisotope. However, $^{3}H$, $123_{I}$, $125_{I}$, $^{131}I$, $^{11}C$, and $^{18}F$ are preferred. Those skilled in the art are familiar with the procedure used to introduce a radioisotope into a compound. For example, a compound of Formula I is prepared wherein one carbon atom is $^{11}C$ or $^{14}C$, and is thus a labeled compound.

The compounds of the present invention can be administered to a patient at dosage levels in the range of about 0.1 to about 1,000 mg per day. For a normal human adult having a body weight of about 70 kg, a dosage in the range of about 0.01 to about 100 mg per kilogram of body weight per day is sufficient. The specific dosage used, however, can vary. For example, the dosage can depend on a number of factors including the requirements of the patient, the severity of the condition being treated, and the pharmacological activity of the compound being used. The determination of optimum dosages for a particular patient is well-known to those skilled in the art.

The examples presented below are intended to illustrate particular embodiments of the invention and are not intended to limit the scope of the specification, including the claims, in any manner.

## EXAMPLES

Compounds of Formula I can be prepared by the synthetic routes outlined in Scheme 1. An appropriately substituted aniline (I) and 5-nitroisobenzofuran-1,3-dione (II) or similarly substituted furan yields the corresponding phthalimides (III) when heated in acetic acid. Standard hydrogenation conditions, such as Raney-nickel/DMF, of (III) yields the reduced phthalimide (IV). Reduction of phthalimide (IV) using standard reducing agents, such as lithium aluminum hydride, gives amine (V). Utilizing Buchwald reaction conditions (J. F. Hartwig, *Angew: Chem. Int. Ed.* 1998: 37:2046-2067), (V) can be converted to (VIII) by reacting (V) and various substituted 2-bromobenzoic acid methyl esters (VI) in the presence of cesium carbonate, tris(dibenzylideneacetone)-dipaladium(0), and (S)-(2,2'-bis(di-p-tolylphosphino-1,1'-binaphthyl) (BINAP) (Method A). In addition, (VIII) can also be obtained by reacting (V) with various substituted 2-fluorobenzoic acid methyl esters (VI) in the presence of lithium dimethyl bis(trimethylsilyl)amide (LHMDS) (Method B). Subsequent saponification of (VIII), utilizing standard conditions such as aqueous sodium hydroxide, yields the desired substituted isoindolines of Formula I. In addition, (V) can be converted directly to a compound of Formula I by reacting (V) with 2-fluorobenzoic acid (VII) in the presence of LHMDS.

Scheme 1

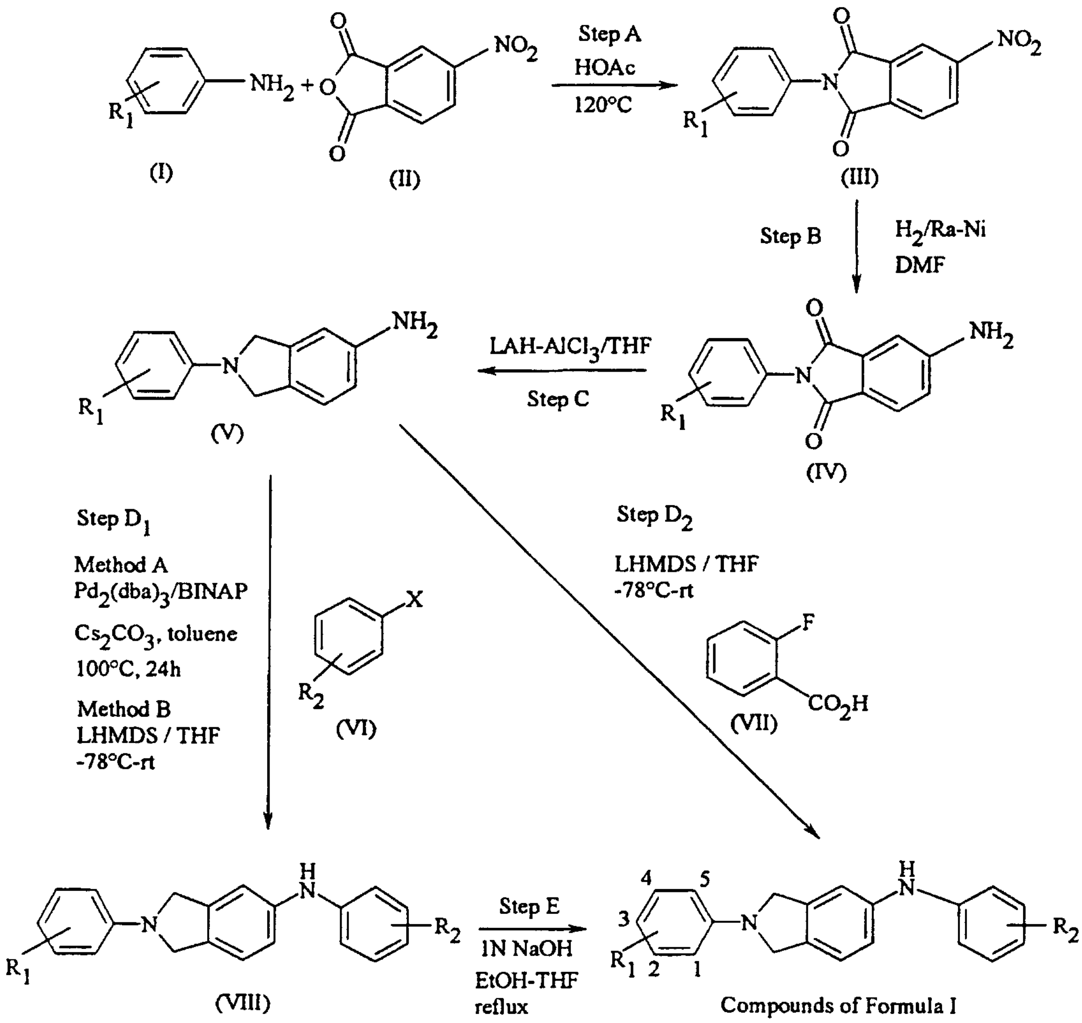


**EXAMPLE 1**

Preparation of 2-[2-(2,3,4-Trimethoxy-phenyl)-2,3-dihydro-1H-isoindol-5-ylamino]-benzoic acid

Step A: Preparation of 5-Nitro-2-(2,3,4-trimethoxy-phenyl)isoindole-1,3-dione

A mixture of 2,3,4-trimethoxyphenylamine (91.61 g, 0.50 mol) and 5-nitroisobenzofuran-1,3-dione (96.56 g, 0.5 mol) in acetic acid (1000 mL) was heated to reflux for 2 hours. After cooling, the precipitate was filtered and washed with $H_2O$ (1 L), 1N NaOH (1.5 L), and $H_2O$ (1 L). The resulting precipitate was triturated with boiling MeOH, filtered and dried in vacuum oven (67°C) for 16 hours to give a yellow solid, 144.0 g (0.40 mol, 80%) of the desired product. Melting point (mp) 245-247°C. Analysis for $C_{17}H_{14}N_2O_4$: Calcd: C, 56.98; H, 3.94; N, 7.82; Found: C, 56.80; H, 4.08; N, 7.80.

Step B: Preparation of 5-Amino-2-(2,3,4-trimethoxy-phenyl)isoindole- 1,3-dione

A sample of 5-nitro-2-(2,3,4-trimethoxy-phenyl)isoindole-1,3-dione (89.0 g, 0.25 mol) in THF (1.25 L), MeOH (1.25 L), and DMF (100 mL) was reduced in the presence of Ra-Ni (12 g) at 28°C to 36°C (ΔP = 7.5 psi). The reaction mixture was filtered, and the filtrate was concentrated in vacuum and dried in vacuum oven (67°C) for 16 hours to give an off-white solid, 81.6 g (0.25 mol, 85%) of the desired product. mp >280°C. Analysis for $C_{17}H_6N_2O_5$: Calcd: C, 62.09;

H, 4.92; N, 8.52; Found: C, 61.71; H, 4.94; N, 8.48.

Step C: Preparation of 2-(2,3,4-Trimethoxyphenyl)-2,3-dihydro-1H-isoindol-5-ylamine

A solution of $AlCl_3$ (8.12 g, 60.92 mmol) in THF (200 mL) was added slowly to a suspension of $LiAlH_4$ (1M/THF, 183 mL, 182 75 mmol) in THF (100 mL) at -40°C and allowed to stir for 10 minutes. The temperature was raised to 0°C and a suspension of 5-amino-2-(2,3,4-trimethoxyphenyl)-isoindole-1,3-dione (20.0 g, 60.92 mmol) in THF (500 mL) was slowly added portionwise. The reaction mixture was stirred for 3 hours while the temperature was warmed to room temperature (rt). The mixture was cooled back to 0°C and carefully quenched with the addition of 25% NaOH (33 mL) and then $H_2O$ (7.3 mL). This mixture was then stirred at rt for 2 hours, filtered through celite, concentrated, and dried in a vacuum oven (60°C) for 16 hours to give a pale solid, 10.8 g (35.96 mmol, 59%) of the desired product mp:213-215°C. Analysis for $C_{17}H_{20}N_2O_3 \cdot 0.14\ H_2O$: Calcd: C, 67.41; H, 6.75; N, 9.25; Found: C, 67.03; H, 6.62; N, 8.98.

Step $D_1$: Preparation of 2-[2-(2,3,4-Trimethoxyphenyl)-2,3-dihydro-1H-isoindol-5-ylamino]benzoic acid methyl ester

A mixture of 2-(2,3,4-trimethoxy-phenyl)-2,3-dihydro-1H-isoindol-5-ylamine (1.2 g, 4.0 mmol), 2-bromo-benzoic acid methyl ester (0.72 g, 3.3 mmol), cesium carbonate (1.52 g, 4.7 mmol), tris(dibenzylideneacetone-dipaladium (0) (91 mg, 0.1 mmol), and (S)-(2,2'-bis(di-p-tolylphosphino-1,1'-binaphthyl (98%, (S)-tol-BINAP) (102 mg, 0.15 mmol) (L/Pd = 1.5) in anhydrous toluene (30 mL) was heated to 100°C for 24 hours under $N_2$. After cooling, the reaction mixture was diluted with ether, filtered through celite, and rinsed thoroughly with ether. The filtrate was evaporated to dryness to give a brown residue. Purification by flash chromatography (silica gel, 10% EtOAc/Hexane) yielded 1.39 g (3.2 mmol, 80%) of the desired product. mp 128-129°C. Analysis for $C_{25}H_{26}N_2O_5$: Calcd: C, 69.11; H, 6.03; N, 6.45; Found: C, 69.39; H, 5.99; N, 6.13.

Step E: Preparation of 2-[2-(2,3,4-Trimethoxy-phenyl)-2,3-dihydro-1H-isoindol-5-ylamino]-benzoic acid

A solution of 2-[2-(2,3,4-trimethoxyphenyl)-2,3-dihydro-1H-isoindol-5-ylamino]benzoic acid methyl ester (1.30 g, 2.99 mmol) and 1N NaOH (aq.) (20.0 mL) in EtOH (5.0 mL) and THF (20 mL) was heated to reflux for 16 hours. The solvent was removed in vacuum. The residue was diluted with $H_2O$ and acidified with concentrated HCl to pH 1. The resulting precipitate was collected by filtration and triturated with boiling MeOH-$H_2O$ (4:1) and dried in vacuum oven for 16 hours to give Example 1, a brown solid (0.96 g, 2.28 mmol, 76%). mp 194-195°C. Analysis for $C_{24}H_{24}N_2O_5$: Calcd: C, 68.56; H, 5.75; N, 6.66; Found: C, 68.19; H, 5.57; N, 6.47.

EXAMPLE 2

Preparation of 5-Nitro-2-[2-(3,4,5-trimethoxyphenyl)-2,3-dihydro-1H-isoindol-5-ylamino]benzoic acid

A mixture of 2-(2,3,4-trimethoxyphenyl)-2,3-dihydro-1H-isoindol-5-ylamine (1.81 g, 6.03 mmol), 2-bromo-5-nitro-benzoic acid methyl ester (1.49 g, 6.02 mmol), cesium carbonate (2 75 g, 8.44 mmol), tris(dibenzylideneacetone-dipaladium(0) (166 mg, 0 18 mmol) and (S)-(2,2'-bis(di-p-tolylphosphino-1,1'-binaphthyl (98%, (S)-tol-BINAP) (184 g, 0.27 mmol) (L/Pd = 1.5) in anhydrous toluene (30 mL) was heated to 100°C for 48 hours under $N_2$. After cooling, the reaction mixture was diluted with ether, filtered through celite, and rinsed thoroughly with ether. The filtrate was evaporated to dryness to give a brown residue (6.3 g). The resulted residue was dissolved in EtOH (10 mL) and THF (20 mL), 5N NaOH (aq.) (40 mL) was added, and the mixture was refluxed for 16 hours. The solvent was removed in vacuum. The residue was acidified with concentrated HCl to pH 3. The resulting precipitate was collected by filtration and triturated with boiling MeOH-$H_2O$ (4:1) and dried in a vacuum oven for 16 hours to give Example 2, a yellow solid (1 8 g, 3.87 mmol, 64%). mp >220°C. Analysis for $C_{24}H_{23}N_3O_7 \cdot 0.71\ H_2O$: Calcd C,60.27; H, 5.15; N, 8.79; Found: C, 59.88; H, 4.90; N, 8.42.

EXAMPLE 3

Preparation of 4-Methoxy-5-nitro-2-[2-(3,4,5-trimethoxyphenyl)-2,3-dihydro-1H-isoindol-5-ylamino]benzoic acid

To a cooled (-78°C) solution of 2-(2,3,4-trimethoxyphenyl)-2,3-dihydro-1H-isoindol-5-ylamine (535 mg, 1.78 mmol) in THF (15 mL), LHMDS (3.56 mL, 1 M in THF, 3.56 mmol) was added dropwise. The reaction mixture was allowed to stir at -78°C for 10 minutes. A solution of 2-fluoro-4-methoxy-5-nitro-benzoic acid methyl ester (408 g, 1.78 mmol) in THF (10 mL) was added dropwise, and this solution was stirred for 30 minutes at -78°C. The reaction mixture was allowed to gradually warm to rt and stir for 2 hours under $N_2$ atmosphere. The reaction mixture was diluted with

EtOAc, and acidified with 5N HCl (pH 3). The organic layer was dried ($Na_2SO_4$), filtered, and concentrated in vacuum to yield a brown residue. To a solution of this residue in EtOH (20 mL) and THF (40 mL), 5N NaOH (aq., 50 mL) was added, and the mixture was refluxed for 16 hours. The solvent was removed, and the residue was acidified with concentrated HCl (pH 3). The precipitate was collected by filtration, triturated with boiling MeOH-$H_2O$ (2:1), and dried in a vacuum oven for 16 hours to give Example 3, a pale solid (550 g, 1.11 mmol, 62%). mp >200°C. Analysis for $C_{25}H_{25}N_8 \cdot 0.42\ H_2O$: Calcd. C, 59.09; H, 5.18; N, 8.35; Found: C, 59.30; H, 4.80; N, 8.24.

EXAMPLE 4

Preparation of 2-[2-(3,4-Dichlorophenyl)-2,3-dihydro-1H-isoindol-5-ylamino]benzoic acid

Step A: Preparation of 2-(3,4-Dichlorophenyl)-5-nitro-isoindole-1,3-dione

The title compound was prepared from 3,4-dichlorophenylamine (48.6 g, 0.3 mol) and 5-nitro-isobenzofuran-1,3-dione (57.9 g, 0.3 mol) in acetic acid (50 mL) using the procedure described in Example 1, Step A to yield a yellow solid, 72.6 g (0.22 mol, 72%) of the desired product. mp 210-211 °C. Analysis of $C_{14}H_6N_2O_4Cl_2$: Calcd: C, 49.88; H, 1.79; N, 8.31; Found: C, 49.71; H, 1.87; N, 8.34.

Step B: Preparation of 5-Amino-2-(3,4-dichlorophenyl)-isoindole-1,3-dione

The title compound was prepared from 2-(3,4-dichlorophenyl)-5-nitro-isoindole-1,3-dione (106.9 g, 0.32 mol), Ra-Ni (5 g) in THF (0.8 L) at 20°C to 76°C ($\Delta P$ = 9.5 psi) using the procedure described in Example 1, Step B to yield a yellow solid, 80.0 g (0.26 mol, 81%) of the desired product. mp 255-257°C. Analysis of $C_{14}H_8N_2O_2Cl_2$. Calcd: C, 54.74; H, 2.63; N, 9.12; Found: C, 54.59; H, 2.65; N, 9.57.

Step $D_1$: Preparation of 2-(3,4-dichlorophenyl)-2,3-dihydro-1H-isoindol-5-ylamine

The title compound was prepared from 5-amino-2-(3,4-dichlorophenyl)-isoindole-1,3-dione (15.0 g, 0.049 mol), $AlCl_3$ (6.51 g, 0.049 mol), $LiAlH_4$ (5.56 g, 0.147 mol) in THF (1000 mL) using the procedure described in Example 1, Step C to yield a tan solid, 12.23 g (0 044 mol, 90%) of the desired product. mp 207-209°C. Analysis of $C_{14}H_{12}N_2Cl_2$ Calcd: C, 60.23; H, 4.33; N, 10.03; Found: C, 59.91; H, 4.36; N, 9.90.

Step C: Preparation of 2-[2-(3,4-dichlorophenyl)-2,3-dihydro-1H-isoindol-5-ylamino]benzoic acid

The title compound was prepared from 2-(3,4-dichlorophenyl)-2,3-dihydro-1H-isoindol-5-ylamine (4.04 g, 14.47 mmol), 2-bromo-benzoic acid methyl ester (2.59 g, 12.06 mmol), cesium carbonate (5.50 g, 16.88 mmol), tris (dibenzylideneacetone-dipaladium(0) (331 mg, 0.36 mmol) and (S)-(2,2'-bis(di-p-tolylphosphino-1,1'-binaphthyl (98%, (S)-tol-BINAP) (368 g, 0.54 mmol) (L/Pd = 1.5) in anhydrous toluene (80 mL), and 5N NaOH (aq.) (100 mL) in EtOH (20 mL) and THF (40 mL) using the procedure described in Example 2 to give a brown solid, 3.11 g (7.79 mmol, 54%) of the desired product. mp >210°C. Analysis of $C_{21}H_{16}N_2O_2Cl_2$: Calcd: C, 63.17; H, 4.04; N, 7.02; Cl, 17.76; Found: C, 63.31; H, 3.94; N, 6.73; Cl, 17.75.

EXAMPLE 5

Preparation of 2-[2-(3,4-Dichlorophenyl)-2,3-dihydro-1H-isoindol-5-ylamino]-5-nitro-benzoic acid

The title compound was prepared from 2-(3,4-dichlorophenyl)-2,3-dihydro-1H-isoindol-5-ylamine (3.00 g, 10.75 mmol), 2-bromo-5-nitrobenzoic acid methyl ester (2 67 g, 10.75 mmol), cesium carbonate (4.90 g, 15.04 mmol), tris(dibenzylideneacetone-dipaladium(0) (295 mg, 0.32 mmol), and (S)-(2,2'-bis(di-p-tolylphosphino-1,1'-binaphthyl (98%, (S)-tol-BINAP) (328 g, 0.48 mmol) (L/Pd = 1.5) in anhydrous toluene (80 mL), and 5N NaOH (aq.) (100 mL) in EtOH (40 mL) and THF (100 mL) using the procedure described in Example 2 to give a brown solid, 3.64 g (8.19 mmol, 76%) of the desired product. mp >222°C. Analysis of $C_{21}H_{15}N_3O_4Cl_2 \cdot 0.1H_2O \cdot 0.25MeOH$: Calcd: C, 56.21; H, 3.60; N, 9.25; Found: C, 56.54; H, 3.74; N, 8.86.

EXAMPLE 6

Preparation of 2-[2-(3,4-Dichlorophenyl)-2,3-dihydro-1H-isoindol-5-ylamino]-4-methoxy-5-nitro-benzoic acid

The title compound was prepared from 2-(3,4-dichlorophenyl)-2,3-dihydro-1H-isoindol-5-ylamine (801 mg, 2.87 mmol), LHDMS (5.74 mL, 1 M in THF, 5.74 mmol), 2-flouro-4-methoxy-5-nitro-benzoic acid methyl ester (658 g, 2.87 mmol) in THF (50 mL), and 5N NaOH (aq., 100 mL) in EtOH (20 mL) and THF (40 mL) using the procedure described in Example 3 to give a brown solid, 1.07 g (2.26 mmol, 79%) of the desired product. mp >230°C. Analysis of $C_{22}H_{17}N_3O_5Cl_2$: Calcd: C, 55.71; H, 3.61; N, 8.86; Found: C, 55.43; H, 3.57; N, 8.80.

EXAMPLE 7

Preparation of 2-[2-(3-chlorophenyl)-2,3-dihydro-1H-isoindol-5-ylamino]benzoic acid

Step A: Preparation of 2-(3-chlorophenyl)-5-nitro-isoindole-1,3-dione

A mixture of 3-chloroaniline (31.89 g, 0.25 mol) and 5-nitro-isobenzofuran-1,3-dione (48.28 g, 0.250 mol) in acetic acid (800 mL) was heated to reflux for 4.5 hours (130-140°C). After cooling, the precipitate was filtered and washed with water (650 mL), 1N NaOH (330 mL), and water (500 mL) to give the product as a white solid, 61.01g (0.202 mol, 81%). mp 210-212°C. Analysis of $C_{14}H_6N_2O_4Cl_2$: Calcd: C, 55.56; H, 2.33; N, 9.26; Found: C, 55.78; H, 1.69; N, 9.15.

Step B: Preparation of 5-Amino-2-(3-chlorophenyl)-isoindole-1,3-dione

The title compound was prepared from 2-(3-chloro-phenyl)-5-nitro-isoindole-1,3-dione (60.94 g, 0.20 mol) in DMF (1.2 L) and was reduced in the presence of Ra-Ni (60 g) at 22°C to 28°C ($\Delta P$ = 6.0 psi). The reaction mixture was filtered, and the filtrate was concentrated in vacuum and then dried in vacuum oven (60°C) for 18 hours to yield a green solid, 54.31g (0.20 mol, 99%) of the desired product. mp 203-205°C. Analysis of $C_{14}H_8N_2O_2Cl_2$: Calcd: C, 61.66; H, 3.33; N, 10.27; Found: C, 61.27; H, 3.40; N, 9.98.

Step C: Preparation of 2-(3-chlorophenyl)-2,3-dihydro-1H-isoindol-5-ylamine

A solution of $AlCl_3$ (4.89 g, 0.037 mol) in THF (200 mL) was added slowly to a suspension of $LiAlH_4$ (4.17g, 0.110 mol) in THF (150 mL) at -40°C and allowed to stir for 10 minutes. The temperature was raised to 0°C and stirred for 10 more minutes. A solution of 5-amino-2-(3-chlorophenyl)-isoindole-1,3-dione (10 g, 0.037 mol) in THF (300 mL) was added dropwise. The reaction was stirred for 3 hours while the solution warmed to room temperature. The reaction was cooled back to 0°C and quenched with 30% NaOH (20 mL), followed by $H_2O$ (40 mL). The solution was filtered through celite, concentrated, and dried in a vacuum oven (60°C) for 16 hours to give the product as a light brown solid, 5.24 g (0.021 mol, 58%) of the desired product. mp 178-186°C. Analysis of $C_{14}H_{12}N_2Cl_2 \cdot 0.11$ mol $H_2O$: C, 68.16; H, 5.40; N, 11.36; Found: C, 68.33; H, 5.46; N, 10.96.

Step $D_2$: Preparation of 2-[2-(3-chlorophenyl)-2,3-dihydro-1H-isoindol-5-ylamino]benzoic acid

To the 2-(3-chloro-phenyl)-2,3-dihydro-1H-isoindol-5-ylamine (3 g, 0.012 mol) in THF(100 mL), LHDMS (36.78 mL, 1 M in THF, 36.78 mmol) was added dropwise at -78°C and allowed to stir for 10 minutes at -78°C. 2-Fluorobenzoic acid (1.72 g, .012 mol) in THF (100 mL) was added dropwise and the solution stirred for 1 hour at -78°C and then was allowed to gradually warm to room temperature and stir for 16 hours. The reaction mixture was concentrated, acidified with 3 M HCl (100 mL) and then extracted with $CH_2Cl_2$ (3 $\times$ 150 mL). The organic layer was dried ($Na_2SO_4$), filtered, and concentrated under vacuum to yield a brown solid. The product was dissolved in MeOH (25 mL) and stirred at room temperature for 10 minutes. Water was added to precipitate the residue. The product was filtered and washed with 1:4 MeOH:$H_2O$ (400 mL) to give a brown solid, 1.02 g (2.80 mmol, 23%) of the desired product. mp 209-212°C. Analysis of $C_{22}H_{17}N_3O_5Cl_2 \cdot 0.29$mol$\cdot H_2O$: C, 68.16; H, 4.79; N, 7.57, Found: C, 67.76; H, 4.56; N, 7.83.

EXAMPLE 8

Preparation of 2-[2-(4-chlorophenyl)-2,3-dihydro-1H-isoindol-5-ylamino]benzoic acid

Step A: Preparation of 2-(4-chlorophenyl)-5-nitro-isoindole-1,3-dione

The title compound was prepared from 4-chlorophenylamine (19.14 g, 0.150 mol) and 5-nitro-isobenzofuran-1,3-dione (28.97 g, 0.150 mol) in acetic acid (300 mL) using the procedure described in Example 1, Step A to yield an orange solid, 42.49 g (0.140 mol, 94%) of the desired product. mp 198-200°C. Analysis of $C_{14}H_6N_2O_4Cl_2$: Calcd: C, 55.56; H, 2.33; N, 9.26; Found: C, 55.52; H, 1.74; N, 8.90.

Step B: Preparation of 5-Amino-2-(4-chlorophenyl)-isoindole-1,3-dione

The title compound was prepared from 2-(4-chlorophenyl)-5-nitro-isoindole-1,3-dione (41.68 g, 0.138 mol), Ra-Ni (40 g) DMF (1.0 L) at 20°C to 27°C (ΔP = 28.5 psi) using the procedure described in Example 1, Step B to yield a green solid, 40.13 g (0.147 mol, 107%) of the desired product. mp 0.228-230°C. Analysis of $C_{14}H_8N_2O_2Cl_2$·0.34 mol $H_2O$: C, 60.31; H, 3.50; N, 10.05; Found: C, 59.92; H, 4.05; N, 11.01.

Step C: Preparation of 2-(4-chlorophenyl)-2,3-dihydro-1H-isoindol-5-ylamine

The title compound was prepared from 5-amino-2-(4-chlorophenyl)-isoindole-1,3-dione (10.0 g, 0.037 mol), $AlCl_3$ (4.89 g, 0.037 mol), $LiAlH_4$ (4.17 g, 0.110 mol) in THF (650 mL) using the procedure described in Example 1, Step C to yield a brown solid, 6.39 g (0.026 mol, 71%) of the desired product. mp 204-210°C. Analysis of $C_{14}H_{12}N_2Cl_2$: Calcd: C, 68 72; H, 5.35, N, 11.45; Found: C, 68.54; H, 5.39; N, 11.16.

Step $D_2$: Preparation of 2-[2-(4-chlorophenyl)-2,3-dihydro-1H-isoindol-5-ylamino]benzoic acid

The title compound was prepared from2-(4-chlorophenyl)-2,3 -dihydro-1H-isoindol-5-ylamine (3 g, 0.012 mol), LHDMS (36.78 mL, 1 M in THF, 36.78 mmol), 2-fluorobenzoic acid (1.72 g, .012 mol) in THF (200 mL) using the procedure described in Example 1, Step $D_2$ to yield a brown solid 1.26 g (0.003 mol, 28%) of the desired product. mp 210-212°C. Analysis of $C_{22}H_{17}N_3O_5Cl_2$·0.11 mol $H_2O$: C, 68.76; H, 4.73; N, 7.64; Found: C, 68.39; H, 4.47; N, 7.76.

EXAMPLE 9

Preparation of 2-[2-(3,4-Dimethylphenyl)-2,3-dihydro-1H-isoindol-5-ylamino]benzoic acid

Step A: Preparation of 2-(3,4-dimethylphenyl)-5-nitro-isoindole-1,3-dione

The title compound was prepared from 3,4-dimethylaniline (30.30 g, 0.250 mol) and 5-nitroisobenzofuran-1,3-dione (48.28 g, 0.250 mol) in acetic acid (500 mL) using the procedure described in Example 7, Step A to yield a yellow solid, 63.37 g (0.234 mol, 94%) of the desired product. mp 173-174°C. Analysis of $C_{14}H_6N_2O_4Cl_2$: Calcd. C, 64 86; H, 4 08, N, 9.45; Found: C, 64.87; H, 3.74; N, 9.39.

Step B: Preparation of 5-Amino-2-(3,4-dimethylphenyl)-isoindole-1,3-dione

The title compound was prepared from 2-(3,4-dimethylphenyl)-5-nitro-isoindole-1,3-dione (63.37 g, 0.213 mol), Ra-Ni (50 g) DMF (1.0 L), MeOH (500 mL) at 22°C to 32°C (ΔP = 4.8 psi) using the procedure described in Example 7, Step B to yield a brown solid, 57 67 g (0.217 mol, 100%) of the desired product. mp 215-218°C. Analysis of $C_{14}H_8N_2O_2Cl_2$·0.06 mol $H_2O$: C, 71.87; H, 5.32; N, 10.48; Found: C, 71.49; H, 5.17; N, 10.49.

Step C: Preparation of 2-(3,4-Dimethylphenyl)-2,3-dihydro-1H-isoindol-S-ylamine

The title compound was prepared from 5-amino-2-(3,4-dimethylphenyl)-isoindole-1,3-dione (7.5 g, 0.028 mol), $AlCl_3$ (3.75 g, 0.028 mol), $LiAlH_4$ (3.19 g, 0..084 mol) in THF (725 mL) using the procedure described in Example 7, Step C to yield a light brown solid, 4.69 g (0.020 mol, 70%) of the desired product. mp 152-155°C. Analysis of $C_{14}H_{12}N_2Cl_2$·0.09 mol $H_2O$: C, 80.09; H, 7.64; N, 11.67; Found: C, 79.72; H, 7.45; N, 11.49.

Step $D_2$: Preparation of 2-[2-(3,4-Dimethylphenyl)-2,3-dihydro-1H-isoindol-5-ylamino]benzoic acid

The title compound was prepared from 2-(3,4-dimethylphenyl)-2,3-dihydro-1H-isoindol-5-ylamine (4 g, 0.017 mol), LHDMS (50.34 mL, 1 M in THF, 50.34 mmol), 2-fluoro-benzoic acid (2.35 g, .017 mol) in THF (200 mL) using the procedure described in Example 7, Step $D_2$ to yield a brown solid 2.95 g (0.008 mol, 49%) of the desired product. mp 198-202°C. Analysis of $C_{22}H_{17}N_3O_5Cl_2 \cdot 0.52$ mol $H_2O$: C, 75.11; H, 6.31; N, 7.62; Found: C, 74.73; H, 6.02; N, 7.75.

EXAMPLE 10

Preparation of 2-[2-(4-Chloro-3-trifluoromethylphenyl)-2,3-dihydro-1H-isoindol-5-ylamino]benzoic acid

Step A: Preparation of 2-(4-Chloro-3-trifluoromethylphenyl)-5-nitro-isoindole-1,3-dione

The title compound was prepared from 5-amino-2-chlorobenzotriflouride (48.89 g, 0.250 mol) and 5-nitroisobenzofuran-1,3-dione (48.28 g, 0.250 mol) in acetic acid (500 mL) using the procedure described in Example 7, Step A to yield an off-white solid, 84.4 g (0.222 mol, 90%) of the desired product. mp 165-168°C. Analysis of $C_{14}H_6N_2O_4Cl_2$: Calcd: C, 48.61; H, 1.63; N, 7.56; Found: C, 48.50; H, 1.29; N, 7.37.

Step B: Preparation of 5-Amino-2-(4-Chloro-3-trifluoromethylphenyl)-isoindole-1,3-dione

The title compound was prepared from 2-(4-Chloro-3-trifluoromethylphenyl)-5-nitro-isoindole-1,3-dione (55.88 g, 0.150 mol), Ra-Ni (11.5 g) DMF (1.0 L) at 27°C ($\Delta P$ = 4.5 psi) using the procedure described in Example 7, Step B to yield a yellow solid, 56.60 g (0.166 mol, >100%) of the desired product. mp 198-201 °C. Analysis of $C_{14}H_8N_2O_2Cl_2 \cdot 0.42$ mol $H_2O$: C, 51.73; H, 2.56; N, 8.04; Found: C, 52.12; H, 2.70; N, 7.62.

Step C: Preparation of 2-(4-Chloro-3-trifluoromethylphenyl)-2,3-dihydro-1H-isoindol-5-ylamine

The title compound was prepared from 5-amino-2-(4-chloro-3-trifluoromethylphenyl)-isoindole-1,3-dione (7.5 g, 0.022 mol), $AlCl_3$ (2.94 g, 0.022 mol), $LiAlH_4$ (2.51 g, 0.066 mol) in THF (700 mL) using the procedure described in Example 7, Step C to yield a yellow solid, 5.78 g (0.018 mol, 84%) of the desired product. mp 148-151°C Analysis of $C_{14}H_{12}N_2Cl_2 \cdot 0.26$ mol $H_2O$. C, 56.76; H, 3.98; N, 8.83; Found: C, 56.42; H, 3.79; N, 8.43.

Step $D_2$: Preparation of 2-[2-(4-Chloro-3-trifluoromethylphenyl)-2,3-dihydro-1H-isoindol-5-ylamino]benzoic acid

The title compound was prepared from 2-(4-chloro-3-trifluoromethylphenyl)-2,3-dihydro-1H-isoindol-5-ylamine (1.75 g, 0.006 mol), LHDMS (16.80 mL, 1 M in THF, 16.80 mmol), 2-fluoro-benzoic acid (0.79 g, .006 mol) in THF (200 mL) using the procedure described in Example 7, Step $D_2$ to yield a brown solid 0.690 g (0.002 mol, 28%) of the desired product. mp 229-233°C. Analysis of $C_{22}H_{17}N_3O_5Cl_2 \cdot 0.47$ mol $H_2O$: C, 59.88; H, 3.87; N, 6.35; Found: C, 59.48; H, 3.81; N, 6.60.

EXAMPLE 11

Preparation of 2-[2-(3-Chloro-phenyl)-2,3-dihydro-1H-isoindol-5-ylamino]-5-nitro-benzoic acid

Step $D_2$: Preparation of 2-[2-(3-Chloro-phenyl)-2,3-dihydro-1H-isoindol-5-ylamino]-5-nitro-benzoic acid

To the 2-(3-chloro-phenyl)-2,3-dihydro-1H-isoindol-1H-ylamine from Example 7, Step C (2 g, 0.008 mol) in THF (100 mL), LHDMS (24.51 mL, 1 M in THF, 24.15 mmol) was added dropwise at -78°C and was allowed to stir for 10 minutes at -78°C. 2-Fluouro-5-nitro-benzoic acid (1.51 g, 0.008 mol) in THF (100 mL) was added dropwise and the solution stirred for 1 hour at -78°C and gradually warmed to room temperature and stirred for 16 hours. The reaction was concentrated and acidified with 3 M HCl (100 mL). The resultant solid was filtered and washed with 10% HCl and then dried under vacuum to yield a brown solid. The product was recrystallized using MeOH and water to give a brown solid, 0.250 g (0.0006 mmol, 7.5%) of the desired product mp: Color change at 130°C, did not melt >260°C. Analysis of $C_{21}H_{16}N_3O_4C1 \cdot 0.24$ mol $H_2O$: C, 60.90; H, 4.01; N, 10.15. Found: C, 60.91, H, 4.01; N, 9.75

The following compounds were prepared by the general methods described in the foregoing examples.

EXAMPLE 12

2-[2-(3,4-Dimethyl-phenyl)-2,3-dihydro-1H-isoindol-5-ylamino]-5-nitro-benzoic acid. mp >280°C. Analysis calc for $C_{23}H_{21}N_3O_4$: C, 68.26; H, 5.27; N, 10.38; Found: C, 67.87; H, 5.17; N, 10.29.

EXAMPLE 13

2-(2-Phenyl-2,3-dihydro-1H-isoindol-5-ylamino)-benzoic acid. mp 220-224°C. Analysis calc for $C_{21}H_{18}N_2O_4$: C, 75.97; H, 5.52; N, 8.44; Found: C, 75.59; H, 5.70; N, 8.40.

EXAMPLE 14

2-[2-(3-Chloro-phenyl)-2,3-dihydro-1H-isoindol-5-ylamino]-5-nitro-benzoic acid. mp >260°C. Analysis calc for $C_{21}H_{16}ClN_3$: C, 60.90; H, 4.01; N, 10.15; Found: C, 60.91; H, 4.01; N, 9.75.

EXAMPLE 15

2-[2-(4-Chloro-phenyl)-2,3-dihydro-1H-isoindol-5-ylamino]-5-nitro-benzoic acid. mp >275°C, MS 410 ($MH^+$).

EXAMPLE 16

[2-(3,4-Dichloro-phenyl)-2,3-dihydro-1H-isoindol-5-yl]-[2-(1H-tetrazol-5-yl)-phenyl]-amine was synthesized as described for Example 4 using a tetrazole fluoro intermediate that was synthesized from commercially available 2-fluorobenzonitrile and sodium azide under standard reaction conditions. mp 203-208°C, MS 423 ($M^-$).

EXAMPLE 17

5-Amino-2-[2-(3,4-dichloro-phenyl)-2,3-dihydro-1H-isoindol-5-ylamino]-benzoic acid was prepared by reaction of the nitro derivative from Example 5 with hydrogen gas in the presence of Raney nickel. mp 242-244°C. Analysis calc for $C_{21}H_{17}C_{12}N_3$: Theory: C, 60.08; H, 4.43; N, 9.79; Found: C, 60 44; H, 4.22; N, 9.40.

EXAMPLE 18

5-Nitro-2-(2-phenyl-2,3-dihydro-1H-isoindol-5-ylamino)-benzoic acid. mp >265°C. Analysis calc for $C_{21}H_{17}N_3O_4$: C, 67.19; H, 4.56; N, 11.19; Found: C, 66.98; H, 4.30; N, 10.86.

EXAMPLE 19

2-[2-(4-Chloro-3-trifluoromethyl-phenyl)-2,3-dihydro-1H-isoindol-5-ylamino]-5-nitro-benzoic acid. mp >260°C. Analysis calc for $C_{22}H_{15}ClF_3$: C, 51.55; H, 3.71; N, 8.20; Found: C, 51.15; H, 3.41; N, 8.17.

EXAMPLE 20

2-[2-(3-Fluoro-phenyl)-2,3-dihydro-1H-isoindol-5-ylamino]-5-nitro-benzoic acid. mp >265°C, MS 392 ($M^+$)

EXAMPLE 21

2-[2-(3-Methoxy-phenyl)-2,3-dihydro-1H-isoindol-5-ylamino]-5-nitro-benzoic acid. mp >290°C. Analysis calc for $C_{22}H_{19}N_3O_5$: C, 64.52; H, 4.91; N, 10.12; Found: C, 64.84; H, 4.90; N, 9.72.

EXAMPLE 22

2-[2-(3-Fluoro-phenyl)-2,3-dihydro-1H-isoindol-5-ylamino]-benzoic acid. MS 349 ($M^+$) Analysis calc for $C_{21}H_{17}FN_2$: C, 70.79; H, 5.06; N, 7.86; Found C, 70.41; H, 4.80, N, 7.68.

EXAMPLE 23

2-[2-(3,4-Dichloro-phenyl)-2,3-dihydro-1H-isoindol-5-ylamino]-5-fluoro-benzoic acid. mp 230-236°C, MS 417

($M^+$).

EXAMPLE 24

2-[2-(3,4-Dichloro-phenyl)-2,3-dihydro-1H-isoindol-5-ylamino]-nicotinic acid. mp 213-225°C. Analysis calc for $C_{20}H_{15}Cl_2N_3$: C, 59.80; H, 3.80; N, 10.46; Found: C, 59.41; H, 3.82; N, 10.21.

EXAMPLE 25

2-[2-(3,4,5-Trimethoxy-phenyl)-2,3-dihydro-1H-isoindol-5-ylamino]-benzonitrile. mp 129-131°C. Analysis calc for $C_{24}H_{23}N_3O_3$: C: 71.80; H, 5.77; N, 10.47; Found: C, 72.09; H, 5.64; N, 10.23.

EXAMPLE 26

2-[2-(3-Methoxy-phenyl)-2,3-dihydro-1H-isoindol-5-ylamino]-benzoic acid. mp 206-211°C.

Invention compounds of Formula I can also be prepared utilizing combinatorial methodology. This procedure permits the rapid synthesis of many analogs embraced by Formula I in small quantities for testing purposes, and then the most potent compounds can be synthesized in large quantities by conventional methods. A typical combinatorial synthetic procedure for compounds of Formula II, for example, is carried out by reacting halo substituted benzoate esters with an amine isoindole to form the corresponding arylamino isoindole, followed by saponification to the carboxylic acid of Formula I (e.g., where y is a benzoic acid moiety). The reactions are carried out on 0.15 mmol scale as follows. Solutions of each halo benzoate reactant (0.18 M) in toluene are placed in 2 dram reaction vials. Each amino isoindole reactant is dissolved in anhydrous toluene to give 0.15 M solutions. A Distriman pipet is used to add 1 mL (0.15 mmol, 1 eq) of each halo benzoate solution to the appropriate vials containing 1 mL (0.18 mmol, 1.2 eq) of the amino isoindole reactants. A catalyst solution is prepared by dissolving 0.025 M of $Pd_2(dba)_3$ (dipalladium-tridibenzylidene acetone) and 0.075 M of BINAP (2,2'-bis(diphenylphosphino)-1,1'-binapthyl) in toluene, and 0.25 mL of the catalyst solution is added to each reaction vial. A base, generally cesium carbonate (68 mg, 0.21 mmol, 1.40 eq) is added to each reaction vial, and the vials are capped and placed in a shaker oven and heated at 100°C for 48 hours. The reaction mixtures are then cooled, and the reaction solvents are removed by evaporation. The solid residue is suspended in 400 μL of ethyl acetate and filtered to remove all catalyst. The filtrates are concentrated to dryness by evaporation to provide compounds of Formula I, wherein the benzoic acid portion is esterified (e.g., benzyl or methyl ester). The esters are dissolved in 500 μL of THF/ethanol (1:1 v/v) to which is added 300 μL of 5 M sodium hydroxide. The solutions are shaken for 5 hours at 60°C and then cooled and concentrated to dryness by evaporation of the solvents to provide the desired compounds of Formula I.

The following compounds can also be prepared in accordance with the procedures set forth above:

2-(2-Phenyl-2,3-dihydro-1H-isoindol-5-ylamino)-benzoic acid;
5-Nitro-2-(2-phenyl-2,3-dihydro-1H-isoindol-5-ylamino)-benzoic acid;
2-[2-(3,4-Dichloro-phenyl)-2,3-dihydro-1H-isoindol-5-ylamino]-benzonitrile;
[2-(3,4-Dichloro-phenyl)-2,3-dihydro-1H-isoindol-5-yl]-(2-tetrazol-1-yl-phenyl)-amine;
{2- [2-(3,4-Dichloro-phenyl)-2,3 -dihydro- 1H-isoindol-5-ylamino]-phenyl}-acetic acid;
3-{2-[2-(3,4-Dichloro-phenyl)-2,3-dihydro-1H-isoindol-5-ylamino]-phenyl}-propionic acid;
2-[2-(3,4-Dichloro-phenyl)-2,3-dihydro-1H-isoindol-5-ylamino]-6-nitro-benzoic acid;
2-[2-(3,4-Dichloro-phenyl)-2,3-dihydro-1H-isoindol-5-ylamino]-4-nitro-benzoic acid;
2-[2-(3,4-Dichloro-phenyl)-2,3-dihydro-1H-isoindol-5-ylamino]-3-nitro-benzoic acid;
2-[2-(3,4-Dichloro-phenyl)-2,3-dihydro-1H-isoindol-5-ylamino]-5-methanesulfonyl-benzoic acid;
2-[2-(3,4-Dichloro-phenyl)-2,3-dihydro-1H-isoindol-5-ylamino]-5-sulfamoyl-benzoic acid;
4-[2-(3,4-Dichloro-phenyl)-2,3-dihydro-1H-isoindol-5-ylamino]-isophthalic acid;
3-[2-(3,4-Dichloro-phenyl)-2,3-dihydro-1H-isoindol-5-yiamino]-phthalic acid;
2-[2-(3,4-Dichloro-phenyl)-2,3-dihydro-1H-isoindol-5-ylamino]-5-trifluoromethyl-benzoic acid;
2-[2-(3,4-Dichloro-phenyl)-2,3-dihydro-1H-isoindol-5-ylamino]-5-imidazol-1-yl-benzoic acid;
[2-(3,4-Dichloro-phenyl)-2,3-dihydro-1H-isoindol-5-yl]-(2-tetrazol-1-ylmethyl-phenyl)-amine;
[2-(3,4-Dichloro-phenyl)-2,3-dihydro-1H-isoindol-5-yl]-[2-(2-tetrazol-1-yl-ethyl)-phenyl]-amine;
[2-(3,4-Dichloro-phenyl)-2,3-dihydro-1H-isoindol-5-yl]-(2-(2-tetrazol-1-yl-vinyl)-phenyl]-amine;
2-[2-(3,4-Dichloro-phenyl)-2,3-dihydro-1H-isoindol-5-ylamino]-5-methyl-benzoic acid;
2-[2-(3,4-Dichloro-phenyl)-2,3-dihydro-1H-isoindol-5-ylamino]-3-methyl-benzoic acid;
2-[2-(3,4-Dichloro-phenyl)-2,3-dihydro-1H-isoindol-5-ylamino]-4-nitro-benzoic acid;
2-[2-(3,4-Dichloro-phenyl)-2,3-dihydro-1H-isoindol-5-ylamino]-3,5-dinitro-benzoic acid;

3-[2-(3,4-Dichloro-phenyl)-2,3-dihydro-1H-isoindol-5-ylamino]-2-methyl-benzoic acid;
2-[2-(3,4-Dichloro-phenyl)-2,3-dihydro-1H-isoindol-5-ylamino]-4-methoxy-benzoic acid;
2-[2-(3,4-Dichloro-phenyl)-2,3-dihydro-1H-isoindol-5-ylamino]4-propoxy-benzoic acid;
4-Butoxy-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino]-4-pentyloxy-benzoic acid;
2-[2-(3,4-Dichloro-phenyl)-2,3-dihydro-1H-isoindol-5-ylamino]-4-hexyloxy-benzoic acid;
2-[2-(3,4-Dichloro-phenyl)-2,3-dihydro-1H-isoindol-5-ylamino]-4-heptyloxy-benzoic acid;
2-[2-(3,4-Dichloro-phenyl)-2,3-dihydro-1H-isoindol-5-ylamino]-4-octyloxy-benzoic acid;
2-[2-(3,4-Dichloro-phenyl)-2,3-dihydro-1H-isoindol-5-ylamino]-4-nonyloxy-benzoic acid;
4-Decyloxy-2-[2-(3,4-dichloro-phenyl)-2,3-dihydro- 1H-isoindol-5-ylamino]-benzoic acid;
2-[2-(3,4-Dichloro-phenyl)-2,3-dihydro-1H-isoindol-5-ylamino)-4-isopropoxy-benzoic acid;
2-[2-(4-Chloro-phenyl)-2,3-dihydro-1H-isoindol-5-ylamino]-5-nitro-benzoic acid;
2-[2-(4-Chloro-3-trifluoromethyl-phenyl)-2,3-dihydro-1H-isoindol-5-ylamino]-5-nitro-benzoic acid;
2-(2-Biphenyl-4-yl-2,3-dihydro-1H-isoindol-5-ylamino)-5-nitro-benzoic acid;
2-[2-(3,4-Dimethyl-phenyl)-2,3-dihydro-1 H-isoindol-5-ylamino]-5-nitro-benzoic acid;
2-[2-(3,4-Dichloro-phenyl)-2,3-dihydro-1H-isoindol-5-ylamino]-5-methoxy-benzoic acid;
2-[2-(3,4-Dichloro-phenyl)-2,3-dihydro-1 H-isoindol-5-ylamino]-3-nitro-benzoic acid;
3-Nitro-2-{2-[(4aS,8aR)-4-(octahydro-isoquinolin-2-yl)-phenyl]-2,3-dihydro-1H-isoindol-5-ylamino}-benzoic acid;
2-{2-[(4aS,8aR)-4-(Octahydro-isoquinolin-2-yl)-phenyl]-2,3-dihydro-1H-isoindol-5-ylamino}-benzoic acid;
4-[2-(3,4-Dichloro-phenyl)-2,3-dihydro-1H-isoindol-5-ylamino]-nicotinic acid;
2-[2-(4-Dibutylamino-phenyl)-2,3-dihydro-1H-isoindol-5-ylamino]-benzoic acid;
2-[2-(3-Dibutylamino-phenyl)-2,3-dihydro-1H-isoindol-5-ylamino]-benzoic acid;
2-[2-(3-Bromo-phenyl)-2,3-dihydro-1H-isoindol-5-ylamino]-benzoic acid;
2-[2-(2-Chloro-phenyl)-2,3-dihydro-1H-isoindol-5-ylamino]-benzoic acid;
5-Dibutylamino-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino]-5-methoxy-benzoic acid;
4-[2-(3,4-Dichloro-phenyl)-2,3-dihydro-1H-isoindol-5-ylamino]-isophthalic acid;
2-(2-Biphenyl-4-yl-2,3-dihydro-1H-isoindol-5-ylamino)-benzoic acid;
2-[2-(3,4-Dimethoxy-phenyl)-2,3-dihydro-1H-isoindol-5-ylamino]-benzoic acid;
2-[2-(3,4-Dihydroxy-phenyl)-2,3-dihydro-1H-isoindol-5-ylamino]-benzoic acid;
2-[2-(3,4-Difluoro-phenyl)-2,3-dihydro-1H-isoindol-5-ylamino]-benzoic acid;
2-[2-(3-Fluoro-4-methyl-phenyl)-2,3-dihydro-1H-isoindol-5-ylamino]-benzoic acid;
2-[2-(3,4,5-Trihydroxy-phenyl)-2,3-dihydro-1H-isoindol-5-ylamino]-benzoic acid;
2-[2-(4-Methyl-3-trifluoromethyl-phenyl)-2,3-dihydro-1H-isoindol-5-ylamino]-benzoic acid;
2-[2-(3,5-Dichloro-phenyl)-2,3-dihydro-1H-isoindol-5-ylamino]-benzoic acid;
2-[2-(2,4-Dichloro-phenyl)-2,3-dihydro-1H-isoindol-5-ylamino]-benzoic acid; or
2-[2-(4-Fluoro-3-trifluoromethyl-phenyl)-2,3-dihydro-1H-isoindol-5-ylamino]-benzoic acid.

The compounds of Formula I have been evaluated in standard in vitro and in vivo assays routinely used by those skilled in amyloid research to evaluate compounds having use in preventing formation of amyloid protein and its aggregation, and in determining the potential usefulness in treating diseases associated with amyloid such as Alzheimer's disease. The following assays are well-established as predictive of clinical utility.

**BIOLOGICAL EXAMPLES**

AMYLOID ASSAYS

**BASSR** (**B**eta-**A**myloid **S**elf-**S**eeding **R**adioassay)

An assay for inhibitors of self-seeded amyloid fibril growth

**Materials:**

Stock Solutions:

*Assay Buffer* - 50 mM sodium phosphate, pH 7.5, 100 mM NaCl, 0.02% $NaN_3$, 1 M urea (filter and store at 4°C)
*Soluble A*β*(1-40) peptide* (Bachem, Torrance, CA) - 2.2 mg/mL in deionized $H_2O$ (stored in aliquots at -20°C, keep on ice when thawed) will self-seed after 1 week storage. Typically, the solution should be stored until no lag phase

is seen in the assay.

*$^{125}$I-labeled A*β *(1-40)* - 150K-350K cpm/μL in 100% acetonitrile -0.1% trifluoroacetic acid (TFA) -1% β-mercaptoethanol (aliquots stored at -20°C) $^{125}$I-labeled Aβ(1-40) can be made in accordance with the procedure set forth by H. LeVine, III in *Neurobiol. Aging,* **16**:755 (1995), which is hereby incorporated by reference, or this reagent may be purchased from Amersham, Arlington Heights, Illinois.

*Final assay conditions:* 30 μM soluble Aβ (1-40) in deionized water in assay buffer + 20-50K cpm $^{125}$I-labeled Aβ(1-40) per assay. Compound to be tested is dissolved in dimethylsulfoxide (DMSO), typically 5-50 mM stock, such that the final concentration of DMSO is <1% v/v in the assay.

**Assay:** Reaction mixture for 50 assays (on ice) is comprised of 0.1-0.2 μL of $^{125}$I-labeled *A$^{125}$I-labeled A*β *(1-40)* + 1 μL of soluble *A*β *(1-40)* + 13.5 μL assay buffer per assay. The following are the amounts of the components of the reaction mixture sufficient for 50 assay wells.

5-10 μL $^{125}$I-labeled *A*β (1-40) dried down
675 μL assay buffer
50 μL soluble *A*β (1-40)

Assay Method

1) Prepare reaction mixture above by mixing components and storing on ice.
2) Pipet 14.5 μL of reaction mixture into each of 50 wells on a polypropylene U-bottom 96-well microtiter plate on ice (Costar 3794).
3) Add 1.7 μL, of diluted compound to be tested to each well in a column of eight, including solvent control. Serial 3-fold dilutions from 1 mM (100 μM final) in assay buffer -urea = 7 dilutions + zero. Each 96-well plate can therefore accommodate 11 samples + 1 Congo Red control (0.039-5 μM final in 2-fold steps).
4) Seal the plate with aluminum film (Beckman 538619) and incubate for 10 minutes on ice.
5) Raise the temperature to 37°C and incubate for 3 to 5 hours (depending on the lot of the peptide).
6) Remove the aluminum film and add 200 μL/well of ice cold assay buffer with urea, collecting the radiolabeled fibrils by vacuum filtration through 0.2 μm pore size GVWP filters in 96-well plates (Millipore MAGV N22, Bedford, MA). Determine the radioactivity of the filters using standard methods well-known to those skilled in the art.

**BASST** (**B**eta-**A**myloid **S**elf-**s**eeding, **T**hioflavinT)

An assay for inhibitors of self-seeded amyloid fibril growth

METHODS:

**Materials**

Stock Solutions:

*Assay Buffer - 50* mM sodium phosphate, pH 7.5, 100 mM NaCl, 0.02% $NaN_3$, 1 M urea (filter and store at 4°C).

*Soluble A*β *(1-40)* - 2.2 mg/mL in deionized $H_2O$ (store in aliquots at -20°C, keep on ice when thawed) will self-seed after 1 week storage. Typically, the solution should be stored until no lag phase is seen in the assay.

*Final assay conditions:* 30 μM soluble *A*β (1-40) in deionized water in assay buffer. Compound to be tested is dissolved in DMSO, typically 5-50 mM stock, such that the final concentration of DMSO is <1% v/v in the assay.

**Assay:** Reaction mixture for 50 assays (on ice) comprised of 1 μL of soluble *A*β (1-40) + 13.5 μL assay buffer per assay. The following are the amounts of the components of the reaction mixture that result in each of the 50 assay wells.

50 μL soluble *A*β (1-40)
675 μL assay buffer

Assay Method

1) Prepare the reaction mix above by mixing the components and storing on ice.
2) Pipet 14.5 μL of reaction mixture into each of 50 wells of a polystyrene U-bottom 96-well microtiter plate (Coming

25881-96) on ice.

3) Add 1.7 μL of diluted compound to be tested to each well in a column of eight, including solvent control. Serial 3-fold dilutions from 1 mM (100 μM final) in assay buffer -urea = 7 dilutions + zero. Each 96-well plate can therefore accommodate 11 samples + 1 Congo Red control (0.039-5 μM final in 2-fold steps).

4) Seal the plate with aluminum film and incubate for 10 minutes on ice.

5) Raise the temperature to 37°C and incubate for 3 to 5 hours (depends on the lot of the peptide).

6) Remove the aluminum film and add 250 μL/well of 5 μM thioflavin T (ThT) [T-3516, Sigma-Aldrich] in 50 mM glycine-NaOH, pH 8.5. Read fluorescence on a plate reader (ex = 440 nm/20 nm; em = 485 nm/20 nm) within 5 minutes.

**BAPA** (**B**eta-**A**myloid **P**eptide **A**ggregation

This assay is used to provide a measure of inhibition by a compound against the aggregation behavior of the beta amyloid peptide.

The purpose of this assay is to provide a higher volume method of assaying the amount of beta amyloid aggregation using an endpoint assay based on filtration. In this assay, hexafluoroisopropanol (HFIP) is used to break down the initial amyloid peptide to a monomer state and use a concentration of 33 μM which is high enough so that aggregation will occur at pH 6.0 in several hours.

METHODS:

β-**A**myloid **P**eptide **A**ggregation. pH 6 0 (**BAPA**)

In a 96-well plate (Costar 3794), we add 25 μL 50 mM Phosphate Buffer, pH 6.0, 10 μL 0.5 mg/mL Aβ (1-40) peptide in 20% HFIP + 0.1 μL/assay radioiodinated $^{125}$I Aβ (1-40) [$^{125}$I Aβ(1-40)], and 1 μL of the compound to be tested starting at 50 mM with a concentration of DMSO <1%. Then, we incubate for 2 to 4 hours at room temperature. We stop the reaction with 200 μL of 50 mM phosphate buffer, pH 6.0, and filter it through a 0.2 μm 96-well filter plate (Millipore MAGU N22) We wash the filter plate with 100 μL of the same phosphate buffer. Aggregation was detected on a Microbeta counter after impregnating the filters with Meltilex (1450-441) and is corrected for background

**BATYM ASSAY**

METHODS:

Required Aβ (1-42)(California Peptide) was dried from its hexafluoroisopropanol (HFIP) stock solution. The Aβ (1-42) was dissolved in dimethylsulfoxide (DMSO) and then mixed with phosphate buffered saline (PBS) (pH 7.4). The mixed Aβ (1-42) solution was filtered through a 0.2 μM Omnipore membrane syringe filter (Millipore, Bedford, MA). The compound to be tested in DMSO (50 times concentrate) was put into each well (0.5 μL/well) of a 96-well plate. The Aβ (1-42) solution was added into each well (24.5 μL/well). The plate was centrifuged at 1,000 g for 5 minutes and incubated at 37°C for 1 day (Aβ 1-42; final concentration 100 μM).

After incubation Thioflavin T (ThT) (30 μM) solution in glycine-NaOH buffer (pH 8.5, 50 mM) was added into each well (250 μL/well), fluorescence was measured (ex. 440/20 nm; em 485/20 nm) using a fluorescence plate reader. The inhibitory activity was calculated as the reduction of fluorescence with the following formula:

$$\text{Inhibition (\%)} = \{(F(A\beta)\text{-}F(A\beta\text{+compound})\}/\{F(A\beta)\text{-}F\text{ (solvent+compound)}\} \times 100$$

The $IC_{50}$s (concentration of test compound required to cause a 50% inhibition of aggregation) were calculated by a curve fitting program using the following equation. The data were obtained from two different experiments in triplicate.

$$\text{Inhibition } (x) = 100\text{-}100/\{1 + (x/IC_{50})^n\},$$

x = concentration of tested compound (M),
$IC_{50}$ = (M),
n = Hill coefficient.

Representative compounds of Formula I have exhibited inhibitory activities ($IC_{50}$) ranging from about 1 μM

to greater than 100 μM in the foregoing assays. The results of these assays for specific representative compounds of the present invention are shown in Table 1 below.

TABLE 1

| Amyloid Inhibitory Activity | | | | |
|---|---|---|---|---|
| Example No. | BASSR $IC_{50}$ μM | BASST $IC_{50}$ μM | BATYM $IC_{50}$ μM | BAPA $IC_{50}$ μM |
| 1 | 5, 6, 10, 5 (P) | 2, 1.6 (P) | 11.3 (P) | 72 |
| | 30,20 (Q) | 3, 3 (Q) | 46.4 (Q) | |
| | >100,15 (R) | 15, 15 (R) | 59.7 (R) | |
| 2 | 30,7.5, 10 | 2,2,2 | 14.1 | |
| 3 | 15,6.2,3.1,4,6,4.5, 10,12 | 3, 2.1 | 17.4 | 64 |
| 4 | 12, 11, 11 (P) | 2, 2, 2 (P) | 26.1 (P) | 101 (P) |
| | 15, 15, 15, (Q) | 2 (Q) | 22.2 (Q) | >100, 93 (Q) |
| 5 | 5,4.1,3 | 3,3,3 | 11.4 | |
| 6 | 7, 7, 12 (P) | 1 (P) | 13.5 (P) | 14 |
| | 4, 5 (Q) | 1.5 (Q) | 14.6 (Q) | |
| 7 | 90, 80, >100 | 1.1 | 37.3 | 28 |
| 8 | >100, >100 | 1 | 33.4 | 19 |
| 9 | > 100 (ppt), > 100 | 1.5 | 32.9 | 13 |
| 10 | 100, 58, 55, 70 | 3 | 34.6 | 16 |
| 11 | 2.5,3.8,5.2, 1.2 (P) | 0.8,1 | 12.6 | >100 |
| 12 | > 100 (ppt) | 1.1 | 11.7 | 5 |
| 13 | >100 (P) | 1 (P) | 90.2, 60 (P) | 4, 21 (P) |
| | > 100 (Q) | 8 (Q) | > 100 (Q) | 32 (Q) |
| | > 100 (R) | 30 (R) | > 100 (R) | 11 (R) |
| | 52 (S) | 4 (S) | 79.7 (S) | |
| | 60 (T) | 2 (T) | 83 (T) | |
| 14 | 2.5, 1.2, 3.8, 5.2 | 0.8, 1.0 | 12.6 | >100 |
| 15 | >100, >100 | 0.6 | 32.5 | >100 |
| 16 | > 100, > 100 (ppt) | 1.8 | 29.5 | 86 |
| 17 | 20 (ppt), 80 | 1.5 | 20 | 40 |
| 18 | 7 (ppt), 4, 5 | 0.9,1.0,0.6 | 16.6 | 97 |
| 19 | 1.1 (v-shape) | 1.0 | 12.8 | > 10 |
| | 1 (v-shape) | | | |
| | 1 (v-shape) | | | |
| 20 | >100 | 2 | 11.5 | >60 |
| 21 | 7,2.2,2.0 | 0.7 | 9.64 | >60 |
| 22 | 10,3,3,6.5,8 | 6,2 | 84 | >60 |
| 23 | >100, 50, 70 | 3,2,2 | 34.1 | 62 |
| 24 | 5,18,18 | 0.8, 0.3, 0.3 | | |
| 25 | >100 (ppt), >100, >100 | 15 | >100 | 25 |
| 26 | >100 (ppt} | 10 | 64.9 | >60 |

A letter in parentheses after particular value indicates the lot of the compound tested. For example, 10 (P) indicates that compound tested was from Lot P. If no lot is specified, the lot of the compound was Lot P. P, Q, R, S, and T are different lots of the compound.

The invention compounds have also shown good activity in standard in vivo assays commonly used to evaluate agents to treat diseases related to aggregation of amyloid proteins, especially Alzheimer's disease and other amyloidoses. In one assay, amyloid protein is induced into the spleen of mice by subcutaneous injections of silver nitrate, Freund's complete adjuvant, and an intravenous injection of amyloid enhancing factor. Silver nitrate is administered each day through Day 11. Test compounds are administered to the mice daily starting on Day 1 through Day 11. On Day 12, the animals are sacrificed, and the spleens are removed, histologically prepared, stained with Congo red, and the percent area of the spleen occupied by birefringent, Congo red-stained amyloid is quantitated microscopically.

Another in vivo assay in which the invention compounds have been evaluated uses transgenic mice. The mice bear a human β-amyloid precursor protein transgene with a prior promoter, as described by Hsiao et al, "Correlative Memory Deficits, A β Elevation, and Amyloid Plaques in Transgenic Mice," *Science* 1966;274:99-102. These transgenic mice develop β-amyloid deposits at about 9 months of age. By 15 months, diffuse and compact senile plaques are abundant, primarily in the neocortex, olfactory bulb, and hippocampus. Invention compounds are administered orally to the mice beginning at the age of 8 months (just prior to the onset of amyloid deposits) and continuing for several months (up to about age 14-18 months). The animals are then sacrificed and the brains are removed. The amount of amyloid in the brain is quantitated both histologically and biochemically.

The above data establishes that representative invention compounds are active in standard assays used to measure inhibition of protein aggregation. The compounds exhibit excellent specificity, for example, as shown in the BASST assay, as well as the BATYM and BAPA assays. The compounds are thus useful to clinically inhibit amyloid protein aggregation and to image amyloid deposits for diagnostic use. The compounds will be used in the form of pharmaceutical formulations, and the following examples illustrate typical compositions.

EXAMPLE 27

| Tablet Formulation | |
|---|---|
| Ingredient | Amount |
| Compound of Example 1 | 50 mg |
| Lactose | 80 mg |
| Cornstarch (for mix) | 10 mg |
| Cornstarch (for paste) | 8 mg |
| Magnesium Stearate (1%) | 2 mg |
| | 150 mg |

The compound of Example 1 is mixed with the lactose and cornstarch (for mix) and blended to uniformity to a powder. The cornstarch (for paste) is suspended in 6 mL of water and heated with stirring to form a paste. The paste is added to the mixed powder, and the mixture is granulated. The wet granules are passed through a No. 8 hard screen and dried at 50°C. The mixture is lubricated with 1% magnesium stearate and compressed into a tablet. The tablets are administered to a patient at the rate of 1 to 4 each day for prevention of amyloid protein aggregation and treatment of Alzheimer's disease.

EXAMPLE 28

Parenteral Solution

In a solution of 700 mL of propylene glycol and 200 mL of water for injection is added 20.0 g of Compound No. 9. The mixture is stirred and the pH is adjusted to 5.5 with hydrochloric acid. The volume is adjusted to 1000 mL with water for injection. The solution is sterilized, filled into 5.0 mL ampoules, each containing 2.0 mL (40 mg of Compound No. 8), and sealed under nitrogen. The solution is administered by injection to a patient suffering from medullary carcinoma of the thyroid and in need of treatment.

EXAMPLE 29

Patch Formulation

Ten milligrams of 2-{4-[3-(3,4-dichloro-phenyl)propyl]phenylamino} benzoic acid is mixed with 1 mL of propylene glycol and 2 μg of acrylic-based polymer adhesive containing a resinous cross-linking agent. The mixture is applied to an impermeable backing (30 $cm^2$) and applied to the upper back of a patient for sustained release treatment

of amyloid polyneuropathy.

The invention and the manner and process of making and using it, are now described in such full, clear, concise, and exact terms as to enable any person skilled in the art to which it pertains, to make and use the same. It is to be understood that the foregoing describes preferred embodiments of the present invention and that modifications may be made therein without departing from the spirit or scope of the present invention as set forth in the claims. To particularly point out and distinctly claim the subject matter regarded as invention, the following claims conclude this specification.

## Claims

1. Compounds having the Formula I:

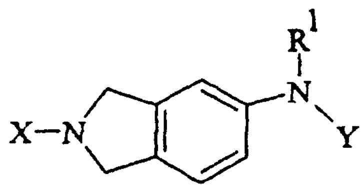

I

or the pharmaceutically acceptable salts thereof,
wherein
X is phenyl or substituted phenyl;
Y is phenyl, substituted phenyl, pyridyl, or substituted pyridyl;
wherein substituted phenyl and substituted pyridyl can have from 1 to 4 substituents, each independently selected from $-OC_1-C_{12}$alkyl, halogen, $-C_1$ $-C_6$alkyl, phenyl,

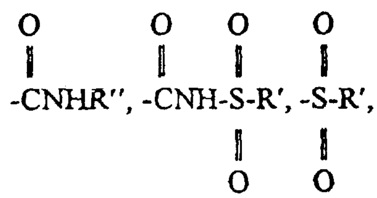

$-CO_2H$, $-CO_2R^1$, $-NO_2$, $-CF_3$, -CN, $-NR^1R^2$, $-(CH_2)_nCO_2H$, $-(CH_2)_nCO_2R^1$, $-SO_2NR^1R^2$, tetrazole, $-(CH_2)_n$-tetrazole, decahydroisoquinoline, imidazole, $-(CH_2)_n$ imidazole, -CH=CH-tetrazole, -CH=CH-imidazole, or phenyl;
$R^1$ and $R^2$ independently are hydrogen or $C_1-C_6$alkyl, and
each n is independently 0 to 5 inclusive.
R" is hydrogen, $C_1-C_6$alkyl, or phenyl; and
R' is hydrogen, $C_1-C_6$alkyl, $-CF_3$, or phenyl.

2. A compound in accordance with Claim 1 wherein
X is substituted phenyl and the substituted phenyl has from 1 to 3 substituents independently selected from $-OC_1-C_6$alkyl, halogen, $C_1-C_6$alkyl, $-CF_3$, or phenyl.

3. A compound in accordance with Claim 1 wherein
Y is substituted phenyl and the substituted phenyl has from 1 to 3 substituents independently selected from $-CO_2H$, $-NO_2$, $-OC_1-C_{12}$ alkyl, -CN, tetrazole, $-(CH_2)_nCO_2H$, $-SO_2NR^1R^2$ $-CF_3$, imidazole, $-(CH_2)_n$-tetrazole, $-(CH_2)_n$ imidazole, -CH=CH-tetrazole, or -CH=CH-imidazole.

4. A compound in accordance with Claim 1 wherein
Y is substituted phenyl and the substituted phenyl has from 1 to 3 substituents, one of which is selected from $-CO_2H$.

5. A compound in accordance with Claim 4 wherein the $-CO_2H$ group is located at the 2-position of the phenyl ring.

**6.** A compound in accordance with Claim 2 wherein the substituted phenyl has two chlorine substituents located at the 3 and 4 positions of the phenyl ring.

**7.** Compounds in accordance with claim 1 having the Formula I:

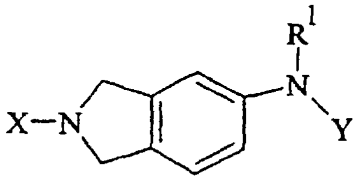

I

or the pharmaceutically acceptable salts thereof,
wherein
X is phenyl or substituted phenyl,
wherein when X is substituted phenyl, the substituted phenyl has from 1 to 4 substituents independently selected from $-OC_1-C_6$alkyl, halogen, $C_1-C_6$alkyl, $-CF_3$, or phenyl;
Y is phenyl or substituted phenyl,
wherein when Y is substituted phenyl, the substituted phenyl has from 1 to 4 substituents independently selected from $-CO_2H$, $-NO_2$, $-OC_1-C_{12}$alkyl, -CN, $-CF_3$, $-(CH_2)_nCO_2H$, $-SO_2NR^1R^2$, tetrazole, $-(CH_2)_n$-tetrazole, imidazole, $-(CH_2)_n$ imidazole, -CH=CH-tetrazole, or -CH=CH-imidazole;
$R^1$ and $R^2$ independently are hydrogen or $C_1-C_6$alkyl; and
each n is independently 0 to 5 inclusive.

**8.** The compounds in accordance whith claim 1:

2-[2-(2,3,4-Trimethoxy-phenyl)-2,3-dihydro-1H-isoindol-5-ylamino]-benzoic acid;
5-Nitro-2-[2-(3,4,5-trimethoxyphenyl)-2,3-dihydro-1H-isoindol-5-ylamino]benzoic acid;
4-Methoxy-5-nitro-2-[2-(3,4,5-trimethoxyphenyl)-2,3-dihydro-1H-isoindol-5-ylamino]benzoic acid;
2-[2-(3,4-Dichlorophenyl)-2,3-dihydro-1H-isoindol-5-ylamino]benzoic acid;
2-[2-(3,4-Dichlorophenyl)-2,3-dihydro-1 H-isoindol-5-ylamino]-5-nitro-benzoic acid,
2-[2-(3,4-Dichlorophenyl)-2,3-dihydro- 1H-isoindol-5-ylamino]-4-methoxy-5-nitro-benzoic acid;
2-[2-(3-Chlorophenyl)-2,3-dihydro-1H-isoindol-5-ylamino]benzoic acid;
2-[2-(4-Chlorophenyl)-2,3-dihydro-1H-isoindol-5-ylamino)benzoic acid;
2-[2-(3,4-Dimethylphenyl)-2,3-dihydro-1H-isoindol-5-ylamino]benzoic acid;
2-[2-(4-Chloro-3-trifluoromethylphenyl)-2,3-dihydro-1H-isoindol-5-ylamino]benzoic acid;
2-[2-Biphenyl-4-yl-2,3-dihydro-1H-isoindol-5-ylamino]benzoic acid; or
2-[2-(3-Chlorophenyl)-2,3-dihydro-1H-isoindol-5-ylamino]-5-nitro-benzoic acid.

**9.** The compounds in accordance with claim 1:

2-(2-Phenyl-2,3-dihydro-1H-isoindol-5-ylamino)-benzoic acid;
5-Nitro-2-(2-phenyl-2,3-dihydro-1H-isoindol-5-ylamino)-benzoic acid;
2-[2-(3,4-Dichloro-phenyl)-2,3-dihydro-1H-isoindol-5-ylamino]-benzonitrile;
[2-(3,4-Dichloro-phenyl)-2,3-dihydro-1H-isoindol-5-yl]-(2-tetrazol-1-yl-phenyl)-amine;
{2-[2-(3,4-Dichloro-phenyl)-2,3-dihydro-1H-isoindol-5-ylamino]-phenyl}-acetic acid;
3-{2-[2-(3,4-Dichloro-phenyl)-2,3-dihydro-1H-isoindol-5-ylamino]-phenyl}-propionic acid;
2-[2-(3,4-Dichloro-phenyl)-2,3-dihydro-1H-isoindol-5-ylamino]-6-nitro-benzoic acid;
2-[2-(3,4-Dichloro-phenyl)-2,3-dihydro-1H-isoindol-5-ylamino]-4-nitro-benzoic acid;
2-[2-(3,4-Dichloro-phenyl)-2,3-dihydro-1H-isoindol-5-ylaminol-3-nitro-benzoic acid;
2-[2-(3,4-Dichloro-phenyl)-2,3-dihydro-1H-isoindol-5-ylamino]-5-methanesulfonyl-benzoic acid;
2-[2-(3,4- Dichloro- phenyl)-2,3 -dihydro-1H-isoindol-5-ylamino]- 5-sulfamoyl-benzoic acid;
4-[2-(3,4-Dichloro-phenyl)-2,3-dihydro-1H-isoindol-5-ylamino]-isophthalic acid;
3-[2-(3,4-Dichloro-phenyl)-2,3-dihydro-1H-isoindol-5-ylamino]-phthalic acid;
2-[2-(3,4-Dichloro-phenyl)-2,3-dihydro-1H-isoindol-5-ylamino]-5-trifluoromethyl-benzoic acid;
2-[2-(3,4-Dichloro-phenyl)-2,3-dihydro-1H-isoindol-5-ylamino]-5-imidazol-1-yl-benzoic acid;
[2-(3,4-Dichloro-phenyl)-2,3-dihydro-1H-isoindol-5-yl]-(2-tetrazol-1-ylmethyl-phenyl)-amine;

[2-(3,4-Dichloro-phenyl)-2,3-dihydro- 1H-isoindol-5-yl]-[2-(2-tetrazol-1-yl-ethyl)-phenyl]-amine
[2-(3,4-Dichloro-phenyl)-2,3-dihydro-1H-isoindol-5-yl]-[2-(2-tetrazol-1-yl-vinyl)-phenyl)-amine;
2-[2-(3,4-Dichloro-phenyl)-2,3-dihydro-1H-isoindol-5-ylamino]-5-methyl-benzoic acid; or
2-[2-(3,4-Dichloro-phenyl)-2,3-dihydro-1H-isoindol-5-ylamino]-3-methyl-benzoic acid.

**10.** The compounds in accordance with claim 1:

2-[2-(3,4-Dichloro-phenyl)-2,3-dihydro-1H-isoindol-5-ylamino]-4-nitro-benzoic acid;
2-[2-(3,4-Dichloro-phenyl)-2,3-dihydro-1H-isoindol-5-ylamino]-3,5-dinitro-benzoic acid;
3-[2-(3,4-Dichloro-phenyl)-2,3-dihydro-1H-isoindol-5-ylamino]-2-methyl-benzoic acid;
2-[2-(3,4-Dichloro-phenyl)-2,3-dihydro-1H-isoindol-5-ylamino]-4-methoxy-benzoic acid;
2-[2-(3,4-Dichloro-phenyl)-2,3-dihydro-1H-isoindol-5-ylamino]-4-propoxy-benzoic acid;
4-Butoxy-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino]-4-pentyloxy-benzoic acid;
2-[2-(3,4-Dichloro-phenyl)-2,3-dihydro-1H-isoindol-5-ylamino]-4-hexyloxy-benzoic acid,
2-[2-(3,4-Dichloro-phenyl)-2,3 -dihydro-1H-isoindol-5-ylamino]-4-heptyloxy-benzoic acid;
2-[2-(3, 4-Dichloro-phenyl)-2,3-dihydro-1H-isoindol-5-ylamino]-4-octyloxy-benzoic acid;
2-[2-(3,4-Dichloro-phenyl)-2,3-dihydro-1H-isoindol-5-ylamino]-4-nonyloxy-benzoic acid;
4-Decyloxy-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino)-4-isopropoxy-benzoic acid;
2-[2-(4-Chloro-phenyl)-2,3-dihydro-1H-isoindol-5-ylamino]-5-nitro-benzoic acid;
2-[2-(4-Chloro-3-trifluoromethyl-phenyl)-2,3-dihydro-1H-isoindol-5-ylamino]-5-nitro-benzoic acid;
2-(2-Biphenyl-4-yl-2,3-dihydro-1H-isoindol-5-ylamino)-5-nitro-benzoic acid; or
2-[2-(3,4-Dimethyl-phenyl)-2,3-dihydro-1H-isoindol-5-ylamino]-5-nitro-benzoic acid.

**11.** The compounds in accordance with claim 1:

2-[2-(3,4-Dimethyl-phenyl)-2,3-dihydro-1H-isoindol-5-ylamino]-5-nitro-benzoic acid;
2-(2-Phenyl-2,3-dihydro-1H-isoindol-5-ylamino)-benzoic ac
2-[2-(3-Chloro-phenyl)-2,3-dihydro-1H-isoindol-5-ylamino]-5-nitro-benzoic acid;
2-[2-(4-Chloro-phenyl)-2,3-dihydro-1H-isoindol-5-ylamino]-5-nitro-benzoic acid
[2-(3,4-Dichloro-phenyl)-2,3-dihydro-1H-isoindol-5-yl]-[2-(1H-tetrazol-5-yl)-phenyl]-amine;
5-Amino-2-[2-(3,4-dichloro-phenyl)-2,3-dihydro-1H-isoindol-5-ylamino]-benzoic acid;
5-Nitro-2-(2-phenyl-2,3-dihydro- 1H-isoindol-5-ylamino)-benzoic acid;
2-[2-(4-Chloro-3-trifluoromethyl-phenyl)-2,3-dihydro-1H-isoindol-5-ylamino]-5-nitro-benzoic acid,
2-[2-(3-Fluoro-phenyl)-2,3-dihydro-1H-isoindol-5-ylaminol-5-nitro-benzoic acid;
2-[2-(3-Methoxy-phenyl)-2,3-dihydro-1H-isoindol-5-ylamino]-5-nitro-benzoic acid;
2-[2-(3-Fluoro-phenyl)-2,3-dihydro- 1H-isoindol-5-ylamino]-benzoic acid;
2-[2-(3,4-Dichloro-phenyl)-2,3-dihydro-1H-isoindol-5-ylamino]-5-fluoro-benzoic acid; and
2-[2-(3,4-Dichloro-phenyl)-2,3-dihydro-1H-isoindol-5-ylamino]-nicotinic acid.

**12.** The compounds in accordance with claim 1:

2-[2-(3,4-Dichloro-phenyl)-2,3-dihydro-1H-isoindol-5-ylamino]-5-methoxy-benzoic acid;
2-[2-(3,4-Dichloro-phenyl)-2,3-dihydro-1H-isoindol-5-ylamino]-3-nitro-benzoic acid;
3-Nitro-2-{2-[(4aS,8aR)-4-(octahydro-isoquinolin-2-yl)-phenyl]-2,3-dihydro-1H-isoindol-5-ylamino}-benzoic acid;
2-{2-[(4aS,8aR)-4-(Octahydro-isoquinolin-2-yl)-phenyl]-2,3-dihydro-1H-isoindol-5-ylamino}-benzoic acid;
4-[2-(3,4-Dichloro-phenyl)-2,3-dihydro-1H-isoindol-5-ylamino]-nicotinic acid;
2-[2-(4-Dibutylamino-phenyl)-2,3-dihydro-1H-isoindol-5-ylamino]-benzoic acid;
2-[2-(3-Dibutylamino-phenyl)-2,3-dihydro-1H-isoindol-5-ylamino]-benzoic acid;
2-[2-(3-Bromo-phenyl)-2,3-dihydro-1H-isoindol-5-ylamino]-benzoic acid,
2-[2-(2-Chloro-phenyl}-2,3-dihydro-1H-isoindol-5-ylamino]-benzoic acid;
5-Dibutylamino-2-[2-(3,4-dichloro-phenyl)-2,3-dihydro-1H-isoindol-5-ylamino]-benzoic acid;
2-[2-(3,4-Dichloro-phenyl)-2,3-dihydro-1H-isoindol-5-ylamino]-5-methoxy-benzoic acid;
4-[2-(3,4-Dichloro-phenyl)-2,3-dihydro-1H-isoindol-5-ylamino]-isophthalic acid;
2-(2-Biphenyl-4-yl-2,3-dihydro-1H-isoindol-5-ylamino)-benzoic acid;
2-[2-(3,4-Dimethoxy-phenyl)-2,3-dihydro-1H-isoindol-5-ylamino]-benzoic acid;

2-[2-(3,4-Dihydroxy-phenyl)-2,3-dihydro-1H-isoindol-5-ylamino]-benzoic acid;
2-[2-(3,4-Dinuoro-phenyl)-2,3-dihydro-1H-isoindol-5-ylamino]-benzoic acid;
2-[2-(3-Fluoro-4-methyl-phenyl)-2,3-dihydro-1H-isoindol-5-ylamino]-benzoic acid;
2-[2-(3,4,5-Trihydroxy-phenyl)-2,3-dihydro-1H-isoindol-5-ylamino]-benzoic acid;
2-[2-(4-Methyl-3-trifluoromethyl-phenyl)-2,3-dihydro-1H-isoindol-5-ylamino]-benzoic acid;
2-[2-(3,5-Dichloro-phenyl)-2,3-dihydro-1H-isoindol-5-ylamino]-benzoic acid;
2-[2-(2,4-Dichloro-phenyl)-2,3-dihydro-1H-isoindol-5-ylamino]-benzoic acid;
2-[2-(4-Fluoro-3-trifluoromethyl-phenyl)-2,3-dihydro-1H-isoindol-5-ylamino]-benzoic acid,
2-[2-(3,4,5-Trimethoxy-phenyl)-2,3-dihydro-1H-isoindol-5-ylamino]-benzonitrile; or
2-[2-(3-Methoxy-phenyl)-2,3-dihydro-1H-isoindol-5-ylamino]-benzoic acid

**13.** A pharmaceutical composition comprising a compound of Claim 1 together with a pharmaceutically acceptable carrier, diluent, or excipient therefor.

**14.** Use of a compound according to claims 1 to 12 for the manufacturing of pharmaceuticals for the treatment of Alzheimer's disease or the inhibition of the aggregation of amyloid proteins to form amyloid deposits.

**15.** Use of a labeled compound according to claims 1 to 12 for the manufacturing of diagnostics for the imaging and detection of amyloid deposits.

**Patentansprüche**

**1.** Verbindungen der Formel I:

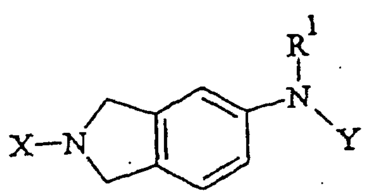

I

oder die pharmazeutisch akzeptablen Salze derselben,
worin
X Phenyl oder substituiertes Phenyl bedeutet;
Y Phenyl, substituiertes Phenyl, Pyridyl oder substituiertes Pyridyl bedeutet;
wobei substituiertes Phenyl und substituiertes Pyridyl 1 bis 4 Substituenten aufweisen können, die jeweils unabhängig voneinander aus $-OC_1-C_{12}$-Alkyl, Halogen, $-C_1-C_6$-Alkyl, Phenyl,

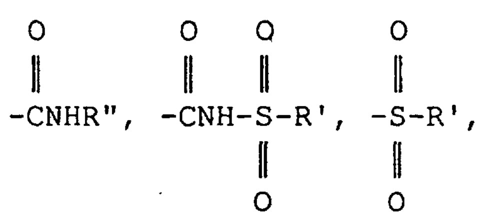

$-CO_2H$, $-CO_2R^1$, $-NO_2$, $-CF_3$, -CN, $-NR^1R^2$, $-(CH_2)_nCO_2H$, $-(CH_2)_nCO_2R^1$, $-SO_2NR^1R^2$, Tetrazol, $-(CH_2)_n$-Tetrazol, Decahydroisochinolin, Imidazol, $-(CH_2)_n$-Imidazol, -CH=CH-Tetrazol, -CH=CH-Imidazol oder Phenyl ausgewählt sind;
$R^1$ und $R^2$ unabhängig voneinander Wasserstoff oder $C_1-C_6$-Alkyl bedeuten; und
jedes n unabhängig voneinander 0 bis einschließlich 5 bedeutet;
R" Wasserstoff, $C_1-C_6$-Alkyl oder Phenyl bedeutet; und
R' Wasserstoff, $C_1-C_6$-Alkyl, $-CF_3$ oder Phenyl bedeutet.

**2.** Verbindung nach Anspruch 1, worin X substituiertes Phenyl bedeutet und das substituierte Phenyl 1 bis 3 Substituenten aufweist, die unabhängig voneinander aus $-OC_1-C_6$-Alkyl, Halogen, $C_1-C_6$-Alkyl, $-CF_3$ oder Phenyl ausgewählt sind.

**3.** Verbindung nach Anspruch 1, worin Y substituiertes Phenyl bedeutet und das substituierte Phenyl 1 bis 3 Substituenten aufweist, die unabhängig voneinander aus $-CO_2H$, $-NO_2$, $-OC_1-C_{12}$-Alkyl, -CN, Tetrazol, $-(CH_2)_nCO_2H$, $-SO_2NR^1R^2$, $-CF_3$, Imidazol, $-(CH_2)_n$-Tetrazol, $-(CH_2)_n$-Imidazol, -CH=CH-Tetrazol oder -CH=CH-Imidazol ausgewählt sind.

**4.** Verbindung nach Anspruch 1, worin Y substituiertes Phenyl bedeutet und das substituierte Phenyl 1 bis 3 Substituenten aufweist, von denen einer aus $-CO_2H$ ausgewählt ist.

**5.** Verbindung nach Anspruch 4, worin die $-CO_2H$-Gruppe an der 2-Position des Phenylrings positioniert ist.

**6.** Verbindung nach Anspruch 2, worin das substituierte Phenyl zwei Chlorsubstituenten, die an der 3- und 4-Position des Phenylrings positioniert sind, aufweist.

**7.** Verbindungen nach Anspruch 1 der Formel I:

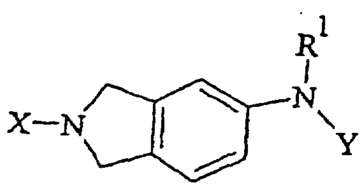

I

oder die pharmazeutisch akzeptablen Salze derselben,
worin
X Phenyl oder substituiertes Phenyl bedeutet,
wobei, wenn X substituiertes Phenyl bedeutet, das substituierte Phenyl 1 bis 4 Substituenten aufweist, die unabhängig voneinander aus $-OC_1-C_6$-Alkyl, Halogen, $C_1-C_6$-Alkyl, $-CF_3$ oder Phenyl ausgewählt sind;
Y Phenyl oder substituiertes Phenyl bedeutet,
wobei, wenn Y substituiertes Phenyl bedeutet, das substituierte Phenyl 1 bis 4 Substituenten aufweist, die unabhängig voneinander aus $-CO_2H$, $-NO_2$, $-OC_1-C_{12}$-Alkyl, -CN, $-CF_3$, $-(CH_2)_nCO_2H$, $-SO_2NR^1R^2$, Tetrazol, $-(CH_2)_n$-Tetrazol, Imidazol, $-(CH_2)_n$-Imidazol, -CH=CH-Tetrazol oder -CH=CH-Imidazol ausgewählt sind;
$R^1$ und $R^2$ unabhängig voneinander Wasserstoff oder $C_1-C_6$-Alkyl bedeuten; und
jedes n unabhängig voneinander 0 bis einschließlich 5 bedeutet.

**8.** Verbindungen nach Anspruch 1, nämlich:

2-[2-(2,3,4-Trimethoxy-phenyl)-2,3-dihydro-1H-isoindol-5-ylamino]-benzoesäure;
5-Nitro-2-[2-(3,4,5-trimethoxyphenyl)-2,3-dihydro-1Hisoindol-5-ylamino]-benzoesäure;
4-Methoxy-5-nitro-2-[2-(3,4,5-trimethoxyphenyl)-2,3-dihydro-1H-isoindol-5-ylamino]-benzoesäure;
2-[2-(3,4-Dichlorphenyl)-2,3-dihydro-1H-isoindol-5-ylamino]-benzoesäure;
2-[2-(3,4-Dichlorphenyl)-2,3-dihydro-1H-isoindol-5-ylamino]-5-nitro-benzoesäure;
2-[2-(3,4-Dichlorphenyl)-2,3-dihydro-1H-isoindol-5-ylamino]-4-methoxy-5-nitro-benzoesäure;
2-[2-(3-Chlorphenyl)-2,3-dihydro-1H-isoindol-5-ylamino]-benzoesäure;
2-[2-(4-Chlorphenyl)-2,3-dihydro-1H-isoindol-5-ylamino]-benzoesäure;
2-[2-(3,4-Dimethylphenyl)-2,3-dihydro-1H-isoindol-5-ylamino]-benzoesäure;
2-[2-(4-Chlor-3-trifluormethylphenyl)-2,3-dihydro-1Hisoindol-5-ylamino]-benzoesäure;
2-[2-Biphenyl-4-yl-2,3-dihydro-1H-isoindol-5-ylamino]-benzoesäure oder
2-[2-(3-Chlorphenyl)-2,3-dihydro-1H-isoindol-5-ylamino]-5-nitro-benzoesäure.

**9.** Verbindungen nach Anspruch 1, nämlich:

2-(2-Phenyl-2,3-dihydro-1H-isoindol-5-ylamino)-benzoesäure;

5-Nitro-2-(2-Phenyl-2,3-dihydro-1H-isoindol-5-ylamino)-benzoesäure;
2-[2-(3,4-Dichlor-phenyl)-2,3-dihydro-1H-isoindol-5-ylamino]-benzonitril;
[2-(3,4-Dichlor-phenyl)-2,3-dihydro-1H-isoindol-5-yl]-(2-tetrazol-1-yl-phenyl)-amin;
{2-[2-(3,4-Dichlor-phenyl)-2,3-dihydro-1H-isoindol-5-ylamino]-phenyl)-essigsäure;
3-{2-[2-(3,4-Dichlor-phenyl)-2,3-dihydro-1H-isoindol-5-ylamino]-phenyl}-propionsäure;
2-[2-(3,4-Dichlor-phenyl)-2,3-dihydro-1H-isoindol-5-ylamino]-6-nitro-benzoesäure;
2-[2-(3,4-Dichlor-phenyl)-2,3-dihydro-1H-isoindol-5-ylamino]-4-nitro-benzoesäure;
2-[2-(3,4-Dichlor-phenyl)-2,3-dihydro-1H-isoindol-5-ylamino]-3-nitro-benzoesäure;
2-[2-(3,4-Dichlor-phenyl)-2,3-dihydro-1H-isoindol-5-ylamino]-5-methansulfonyl-benzoesäure;
2-[2-(3,4-Dichlor-phenyl)-2,3-dihydro-1H-isoindol-5-ylamino]-5-sulfamoyl-benzoesäure;
4-[2-(3,4-Dichlor-phenyl)-2,3-dihydro-1H-isoindol-5-ylamino]-isophthalsäure;
3-[2-(3,4-Dichlor-phenyl)-2,3-dihydro-1H-isoindol-5-ylamino]-phthalsäure;
2-[2-(3,4-Dichlor-phenyl)-2,3-dihydro-1H-isoindol-5-ylamino]-5-trifluormethyl-benzoesäure;
2-[2-(3,4-Dichlor-phenyl)-2,3-dihydro-1H-isoindol-5-ylamino]-5-imidazol-1-yl-benzoesäure;
[2-(3,4-Dichlor-phenyl)-2,3-dihydro-1H-isoindol-5-yl)-(2-tetrazol-1-ylmethyl-phenyl)-amin;
[2-(3,4-Dichlor-phenyl)-2,3-dihydro-1H-isoindol-5-yl]-[2-(2-tetrazal-1-yl-ethyl)-phenyl]-amin;
[2-(3,4-Dichlor-phenyl)-2,3-dihydro-1H-isoindol-5-yl]-[2-(2-tetrazol-1-yl-vinyl)-phenyl]-amin;
2-[2-(3,4-Dichlor-phenyl)-2,3-dihydro-1H-isoindol-5-ylamino]-5-methyl-benzoesäure oder
2-[2-(3,4-Dichlor-phenyl)-2,3-dihydro-1H-isoindol-5-ylamino]-3-methyl-benzoesäure.

**10.** Verbindungen nach Anspruch 1, nämlich:

2-[2-(3,4-Dichlor-phenyl)-2,3-dihydro-1H-isoindol-5-ylamino]-4-nitro-benzoesäure;
2-[2-(3,4-Dichlor-phenyl)-2,3-dihydro-1H-isoindol-5-ylamino]-3,5-dinitro-benzoesäure;
3-[2-(3,4-Dichlor-phenyl)-2,3-dihydro-1H-isoindol-5-ylamino]-2-methyl-benzoesäure;
2-[2-(3,4-Dichlor-phenyl)-2,3-dihydro-1H-isoindol-5-ylamino]-4-methoxy-benzoesäure;
2-[2-(3,4-Dichlor-phenyl)-2,3-dihydro-1H-isoindol-5-ylamino]-4-propoxy-benzoesäure;
4-Butoxy-2-[2-(3,4-dichlor-phenyl)-2,3-dihydro-1H-isoindol-5-ylamino]-benzoesäure;
2-[2-(3,4-Dichlor-phenyl)-2,3-dihydro-1H-isoindol-5-ylamino]-4-pentyloxy-benzoesäure;
2-[2-(3,4-Dichlor-phenyl)-2,3-dihydro-1H-isoindol-5-ylamino]-4-hexyloxy-benzoesäure;
2-[2-(3,4-Dichlor-phenyl)-2,3-dihydro-1H-isoindol-5-ylamino]-4-heptyloxy-benzoesäure;
2-[2-(3,4-Dichlor-phenyl)-2,3-dihydro-1H-isoindol-5-ylamino]-4-octyloxy-benzoesäure;
2-[2-(3,4-Dichlor-phenyl)-2,3-dihydro-1H-isoindol-5-ylamino]-4-nonyloxy-benzoesäure;
4-Decyloxy-2-[2-(3,4-dichlor-phenyl)-2,3-dihydro-1Hisoindol-5-ylamino]-benzoesäure;
2-[2-(3,4-Dichlor-phenyl)-2,3-dihydro-1H-isoindol-5-ylamino]-4-isopropoxy-benzoesäure;
2-[2-(4-Chlor-phenyl)-2,3-dihydro-1H-isoindol-5-ylamino]-5-nitro-benzoesäure;
2-[2-(4-Chlor-3-trifluormethyl-phenyl)-2,3-dihydro-1Hisoindol-5-ylamino]-5-nitro-benzoesäure;
2-(2-Biphenyl-4-yl-2,3-dihydro-1H-isoindol-5-ylamino]-5-nitro-benzoesäure oder
2-[2-(3,4-Dimethyl-phenyl)-2,3-dihydro-1H-isoindol-5-ylamino]-5-nitro-benzoesäure.

**11.** Verbindungen nach Anspruch 1, nämlich:

2-[2-(3,4-Dimethyl-phenyl)-2,3-dihydro-1H-isoindol-5-ylamino]-5-nitro-benzoesäure;
2-(2-Phenyl-2,3-dihydro-1H-isoindol-5-ylamino)-benzoesäure;
2-[2-(3-Chlor-phenyl)-2,3-dihydro-1H-isoindol-5-ylamino]-5-nitro-benzoesäure;
2-[2-(4-Chlor-phenyl)-2,3-dihydro-1H-isoindol-5-ylamino]-5-nitro-benzoesäure;
[2-(3,4-Dichlor-phenyl)-2,3-dihydro-1H-isoindol-5-yl][2-(1H-tetrazol-5-yl)-phenyl]-amin;
5-Amino-2-[2-(3,4-Dichlor-phenyl)-2,3-dihydro-1H-isoindol-5-ylamino]-benzoesäure;
5-Nitro-2-(2-phenyl-2,3-dihydro-1H-isoindol-5-ylamino)-benzoesäure;
2-[2-(4-Chlor-3-trifluormethyl-phenyl)-2,3-dihydro-1Hisoindol-5-ylamino]-5-nitro-benzoesäure;
2-[2-(3-Fluor-phenyl)-2,3-dihydro-1H-isoindol-5-ylamino]-5-nitro-benzoesäure;
2-[2-(3-Methoxy-phenyl)-2,3-dihydro-1H-isoindol-5-ylamino]-5-nitro-benzoesäure;
2-[2-(3-Fluor-phenyl)-2,3-dihydro-1H-isoindol-5-ylamino]-benzoesäure;
2-[2-(3,4-Dichlor-phenyl)-2,3-dihydro-1H-isoindol-5-ylamino]-5-fluor-benzoesäure und
2-[2-(3,4-Dichlor-phenyl)-2,3-dihydro-1H-isoindol-5-ylamino]-nicotinsäure.

**12.** Verbindung nach Anspruch 1, nämlich:

2-[2-(3,4-Dichlor-phenyl)-2,3-dihydro-1H-isoindol-5-ylamino]-5-methoxy-benzoesäure;
2-[2-(3,4-Dichlor-phenyl)-2,3-dihydro-1H-isoindol-5-ylamino]-3-nitro-benzoesäure;
3-Nitro-2-{2-[(4aS,8aR)-4-(octahydro-isochinolin-2-yl)-phenyl]-2,3-dihydro-1H-isoindol-5-ylamino)-benzoesäure;
2-{2-[(4aS,8aR)-4-(Octahydro-isochinolin-2-yl)-phenyl]-2,3-dihydro-1H-isoindol-5-ylamino}-benzoesäure;
4-[2-(3,4-Dichlor-phenyl)-2,3-dihydro-1H-isoindol -5-ylamino]-nicotinsäure;
2-[2-(4-Dibutylamino-phenyl)-2,3-dihydro-1H-isoindol-5-ylamino]-benzoesäure;
2-[2-(3-Dibutylamino-phenyl)-2,3-dihydro-1H-isoindol-5-ylamino]-benzoesäure;
2-[2-(3-Brom-phenyl)-2,3-dihydro-1H-isoindol-5-ylamino]-benzoesäure;
2-[2-(2-Chlor-phenyl)-2,3-dihydro-1H-isoindol-5-ylamino]-benzoesäure;
5-Dibutylamino-2-(2-(3,4-dichlor-phenyl)-2,3-dihydro-1Hisoindol-5-ylamino]-benzoesäure;
2-[2-(3,4-Dichlor-phenyl)-2,3-dihydro-1H-isoindol-5-ylamino]-5-methoxy-benzoesäure;
4-[2-(3,4-Dichlor-phenyl)-2,3-dihydro-1H-isoindol-5-ylamino]-isophthalsäure;
2-(2-Biphenyl-4-yl-2,3-dihydro-1H-isoindol-5-ylamino]-benzoesäure;
2-[2-(3,4-Dimethoxy-phenyl)-2,3-dihydro-1H-isoindol-5-ylamino]-benzoesäure;
2-[2-(3,4-Dihydroxy-phenyl)-2,3-dihydro-1H-isoindol-5-ylamino]-benzoesäure;
2-[2-(3,4-Difluor-phenyl)-2,3-dihydro-1H-isoindol-5-ylamino]-benzoesäure;
2-[2-(3-Fluor-4-methyl-phenyl)-2,3-dihydro-1H-isoindol-5-ylamino]-benzoesäure;
2-[2-(3,4,5-Trihydroxy-phenyl)-2,3-dihydro-1H-isoindol-5-ylamino]-benzoesäure;
2-[2-(4-Methyl-3-trifluormethyl-phenyl)-2,3-dihydro-1Hisoindol-5-ylamino]-benzoesäure;
2-[2-(3,5-Dichlor-phenyl)-2,3-dihydro-1H-isoindol-5-ylamino]-benzoesäure;
2-[2-(2,4-Dichlor-phenyl)-2,3-dihydro-1H-isoindol-5-ylamino]-benzoesäure;
2-[2-(4-Fluor-3-trifluormethyl-phenyl)-2,3-dihydro-1Hisoindol-5-ylamino]-benzoesäure;
2-[2-(3,4,5-Trimethoxy-phenyl)-2,3-dihydro-1H-isoindol-5-ylamino]-benzoitril oder
2- [2- (3-Methoxy-phenyl) -2, 3-dihydro-1H-isoindol-5-ylamino]-benzoesäure.

**13.** Pharmazeutische Zusammensetzung, die eine Verbindung nach Anspruch 1 zusammen mit einem pharmazeutisch akzeptablen Träger, Verdünnungsmittel oder Streckmittel hierfür umfasst.

**14.** Verwendung einer Verbindung nach den Ansprüchen 1 bis 12 zur Herstellung von Arzneimitteln zur Behandlung von Alzheimer-Krankheit oder Hemmung der Aggregation von Amyloidproteinen zur Bildung von Amyloidablagerungen.

**15.** Verwendung einer markierten Verbindung nach den Ansprüchen 1 bis 12 zur Herstellung von diagnostischen Mitteln zur Bildgebung und Detektion von Amyloidablagerungen.

## Revendications

**1.** Composés possédant la formule I :

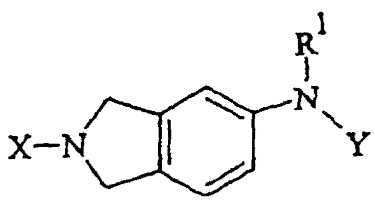

I

ou ses sels pharmaceutiquement acceptables,
dans laquelle
X représente un groupe phényle ou phényle substitué ;
Y représente un groupe phényle, phényle substitué, pyridyle, ou pyridyle substitué ;

où les groupes phényle substitué et pyridyle substitué peuvent avoir de 1 à 4 substituants, chacun indépendamment choisi parmi les groupes -O(alkyle en $C_1$ à $C_{12}$), halogéno, -alkyle en $C_1$ à $C_6$, phényle,

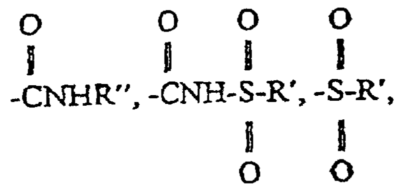

$-CO_2H$, $-CO_2R^1$, $-NO_2$, $-CF_3$, -CN, $-NR^1R^2$, $-(CH_2)_nCO_2H$, $-(CH2)_nCO_2R^1$, $-SO_2NR^1R^2$, tétrazole, $-(CH_2)_n$-tétrazole, décahydroiso-quinoléine, imidazole, $-(CH_2)_n$-imidazole, -CH=CH-tétrazole, -CH=CH-imidazole, ou phényle ;
$R^1$ et $R^2$ représentent, indépendamment l'un de l'autre, un atome d'hydrogène ou un groupe alkyle en $C_1$ à $C_6$ ; et chaque n vaut, indépendamment les un des autres, 0 à 5 inclus ;
R" représente un atome d'hydrogène, un groupe alkyle en $C_1$ à $C_6$, ou phényle ; et
R' représente un atome d'hydrogène, un groupe alkyle en $C_1$ à $C_6$, $-CF_3$, ou phényle.

**2.** Composé selon la revendication 1, où X représente un groupe phényle substitué et le groupe phényle substitué possède de 1 à 3 substituants choisis, indépendamment les uns des autres, parmi les groupes -O(alkyle en $C_1$ à $C_6$), halogéno, alkyle en $C_1$ à $C_6$, $-CF_3$, ou phényle.

**3.** Composé selon la revendication 1, où Y représente un groupe phényle substitué et le groupe phényle substitué possède de 1 à 3 substituants choisis, indépendamment les uns des autres, parmi les groupes $-CO_2H$, $-NO_2$, -O (alkyle en $C_1$ à $C_{12}$), -CN, tétrazole, $-(CH_2)_nCO_2H$, $-SO_2NR^1R^2$, $-CF_3$, imidazole, $-(CH_2)_n$-tétrazole, $-(CH_2)_n$-imidazole, -CH=CH-tétra-zole, ou -CH=CH-imidazole.

**4.** Composé selon la revendication 1, où Y représente un groupe phényle substitué et le groupe phényle substitué possède de 1 à 3 substituants, dont l'un est choisi dans le groupe $-CO_2H$.

**5.** Composé selon la revendication 4, dans lequel le groupe $-CO_2H$ est situé en la position 2 du noyau phényle.

**6.** Composé selon la revendication 2, dans lequel le groupe phényle substitué possède deux substituants chloro situés en positions 3 et 4 du noyau phényle.

**7.** Les composés selon la revendication 1, possédant la formule I :

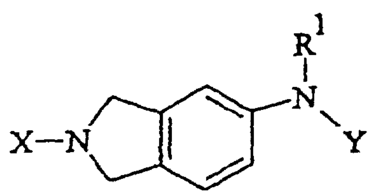

I

ou ses sels pharmaceutiquement acceptables,
dans laquelle
X représente un groupe phényle ou phényle substitué,
où quand X représente un groupe phényle substitué, le groupe phényle substitué possède de 1 à 4 substituants choisis, indépendamment les uns des autres, parmi les groupes -O(alkyle en $C_1$ à $C_6$), halogéno, alkyle en $C_1$ à $C_6$, $-CF_3$, ou phényle ;
Y représente un groupe phényle ou phényle substitué,
où quand Y représente un groupe phényle substitué, le groupe phényle substitué possède de 1 à 4 substituants choisis, indépendamment les un des autres, parmi les groupes $-CO_2H$, $-NO_2$, -O (alkyle en $C_1$ à $C_{12}$), -CN, $-CF_3$, $-(CH_2)_nCO_2H$, $-SO_2NR^1R^2$, tétrazole, $-(CH_2)_n$-tétrazole, imidazole, $-(CH_2)_n$-imidazole, -CH=CH-tétrazole, ou -CH=CH-imidazole ;
$R^1$ et $R^2$ représentent, indépendamment l'un de l'autre, un atome d'hydrogène ou un groupe alkyle en $C_1$ à $C_6$ ; et chaque n vaut, indépendamment les uns des autres, de 0 à 5 inclus.

**8.** Les composés selon la revendication 1 :

acide 2-[2-(2,3,4-triméthoxy-phényle)-2,3-dihydro-1H-isoindol-5-ylamino]-benzoïque ;
acide 5-nitro-2-[2-(3,4,5-triméthoxyphényl)-2,3-dihydro-1H-isoindol-5-ylamino]benzoïque ;
acide 4-méthoxy-5-nitro-2-[2-(3,4,5-triméthoxy-phényl)-2,3-dihydro-1H-isoindol-5-ylamino]benzoïque ;
acide 2-[2-(3,4-dichlorophényl)-2,3-dihydro-1H-isoindol-5-ylamino]benzoïque ;
acide 2-[2-(3,4-dichlorophényl)-2,3-dihydro-1H-isoindol-5-ylamino]-5-nitro-benzoïque ;
acide 2-[2-(3,4-dichlorophényl)-2,3-dihydro-1H-isoindol-5-ylamino]-4-méthoxy-5-nitro-benzoïque ;
acide 2-[2-(3-chlorophényl)-2,3-dihydro-1H-isoindol-5-ylamino]benzoïque ;
acide 2-[2-(4-chlorophényl)-2,3-dihydro-1H-isoindol-5-ylamino]benzoïque ;
acide 2-[2-(3,4-diméthylphényl)-2,3-dihydro-1H-isoindol-5-ylamino]benzoïque ;
acide 2-[2-(4-chloro-3-trifluorométhylphényl)-2,3-dihydro-1H-isoindol-5-ylamino] benzoïque ;
acide 2- [2-biphényl-4-yl-2,3-dihydro-1H-isoindol-5-ylam ino]benzoique ; ou
acide 2-[2-(3-chlorophényl)-2,3-dihydro-1H-isoindol-5-ylamino]-5-nitro-benzoïque.

**9.** Les composés selon la revendication 1 :

acide 2-(2-phényl-2,3-dihydro-1H-isoindol-5-ylamino)-benzoïque ;
acide 5-nitro-2-(2-phényl-2,3-dihydro-1H-isoindol-5-ylamino)-benzoïque ;
2- [2- (3,4-dichloro-phényl) -2,3-dihydro-1H-isoindol-5-ylamino] -benzonitrile ;
[2- (3,4-dichloro-phényl) -2,3-dihydro-1H-isoindol-5-yl]-(2-tétrazol-1-yl-phényl)-amine ;
acide {2-[2-(3,4-dichloro-phényl)-2,3-dihydro-1H-isoindol-5-ylamino] phényl}-acétique ;
acide 3-{2-[2-(3,4-dichloro-phényl)-2,3-dihydro-1H-isoindol-5-ylamino]-phényl}-propionique ;
acide 2-[2-(3,4-dichloro-phényl)-2,3-dihydro-1H-isoindol - 5-ylamino]-6-nitro-benzoique ;
acide 2-[2-(3,4-dichloro-phényl)-2,3-dihydro-1H-isoindol-5-ylamino]-4-nitro-benzoïque ;
acide 2-[2-(3,4-dichloro-phényl)-2,3-dihydro-1H-isoindol-5-ylamino]-3-nitro-benzoïque;
acide 2-[2-(3,4-dichloro-phényl)-2,3-dihydro-1H-isoindol-5-ylamino]-5-méthanesulfonyl-benzoïque ;
acide 2-[2-(3,4-dichloro-phényl)-2,3-dihydro-1H-isoindol-5-ylamino]-5-sulfamoyl-benzoïque ;
acide 4-[2-(3,4-dichloro-phényl)-2,3-dihydro-1H-isoindol-5-ylamino]-isophthalique ;
acide 3-[2-(3,4-dichloro-phényl)-2,3-dihydro-1H-isoindol-5-ylamino] -phthalique ;
acide 2-[2-(3,4-dichloro-phényl)-2,3-dihydro-1H-isoindol-5-ylamino]-5-trifluorométhyl-benzoïque ;
acide 2-[2-(3,4-dichloro-phényl)-2,3-dihydro-1H-isoindol-5-ylamino]-5-imidazol-1-yl-benzoïque ;
[2-(3,4-dichloro-phényl)-2,3-dihydro-1H-isoindol-5-yl]-(2-tétrazol-1-ylméthyl-phényl)-amine ;
[2-(3,4-dichloro-phényl)-2,3-dihydro-1H-isoindol-5-yl]-[2-(2-tétrazol-1-yl-éthyl)-phényl]-amine ;
[2-(3,4-dichloro-phényl)-2,3-dihydro-1H-isoindol-5-yl]-[2-(2-tétrazol-1-yl-vinyl)-phényl]-amine;
acide 2-[2-(3,4-dichloro-phényl)-2,3-dihydro-1H-isoindol-5-ylamino]-5-méthyl-benzoïque ; ou
acide 2-[2-(3,4-dichloro-phényl)-2,3-dihydro-1H-isoindol-5-ylamino]-3-méthyl-benzoïque.

**10.** Les composés selon la revendication 1 :

acide 2-[2-(3,4-dichloro-phényl)-2,3-dihydro-1H-isoindol-5-ylamino]-4-nitro-benzoïque ;
acide 2-[2-(3,4-dichloro-phényl)-2,3-dihydro-1H-isoindol-5-ylamino]-3,5-dinitro-benzoïque ;
acide 3-[2-(3,4-dichloro-phényl)-2,3-dihydro-1H-isoindol-5-ylamino] -2-méthyl-benzoïque ;
acide 2- [2- (3,4-dichloro-phényl) -2,3-dihydro-1H-isoindol-5-ylamino]-4-méthoxy-benzoïque ;
acide 2-[2-(3,4-dichloro-phényl)-2,3-dihydro-1H-isoindol-5-ylamino]-4-propoxy-benzoïque ;
acide 4-butoxy-2-[2-(3,4-dichloro-phenyl)-2,3-dihydro-1H-isoindol-5-ylamino]-benzoïque ;
acide 2-[2-(3,4-dichloro-phényl)-2,3-dihydro-1H-isoindol-5-ylamino]-4-pentyloxy-benzoïque ;
acide 2-[2-(3,4-dichloro-phényl)-2,3-dihydro-1H-isoindol-5-ylamino]-4-hexyloxy-benzoïque ;
acide 2-[2-(3,4-dichloro-phényl)-2,3-dihydro-1H-isoindol-5-ylamino]-4-heptyloxy-benzoïque ;
acide 2-[2-(3,4-dichloro-phényl)-2,3-dihydro-1H-isoindol-5-ylamino]-4-octyloxy-benzoïque ;
acide 2-[2-(3,4-dichloro-phényl)-2,3-dihydro-1H-isoindol-5-ylamino]-4-nonyloxy-benzoïque ;
acide 4-décyloxy-2-[2-(3,4-dichloro-phényl)-2,3-dihydro-1H-isoindol-5-ylamino)-benzoïque ;
acide 2-[2-(3,4-dichloro-phényl)-2,3-dihydro-1H-isoindol-5-ylamino]-4-isopropoxy-benzoïque ;
acide 2-[2-(4-chloro-phényl)-2,3-dihydro-1H-isoindol-5-ylamino]-5-nitro-benzoïque ;
acide 2-[2-(4-chloro-3-trifluorométhyl-phényl)-2,3-dihydro-1H-isoindol-5-ylamino]-5-nitro-benzoïque ;
acide 2-(2-biphényl-4-yl-2,3-dihydro-1H-isoindol-5-ylamino)-5-nitro-benzoïque ; ou
acide 2-[2-(3,4-diméthyl-phényl)-2,3-dihydro-1H-isoindol-5-ylamino]-5-nitro-benzoïque.

**11.** Les composés selon la revendication 1 :

acide 2-[2-(3,4-diméthyl-phényl)-2,3-dihydro-1H-isoindol-5-ylamino]-5-nitro-benzoïque ;
acide 2-(2-phényl-2,3-dihydro-1H-isoindol-5-ylamino)-benzoïque ;
acide 2-[2-(3-chloro-phényl)-2,3-dihydro-1H-isoindol-5-ylamino]-5-nitro-benzoïque ;
acide 2-[2-(4-chloro-phényl)-2,3-dihydro-1H-isoindol-5-ylamino]-5-nitro-benzoïque ;
[2-(3,4-dichloro-phényl)-2,3-dihydro-1H-isoindol-5-yl]-[2-(1H-tétrazol-5-yl)-phényl]-amine ;
acide 5-amino-2-[2-(3,4-dichloro-phényl)-2,3-dihydro-1H-isoindol-5-ylamino]-benzoïque ;
acide 5-nitro-2-(2-phényl-2,3-dihydro-1H-isoindol-5-ylamino)-benzoïque ;
acide 2-[2-(4-chloro-3-trifluorométhyl-phényl)-2,3-dihydro-1H-isoindol-5-ylamino]-5-nitro-benzoïque ;
acide 2-[2-(3-fluoro-phényl)-2,3-dihydro-1H-isoindol-5-ylamino]-5-nitro-benzoïque ;
acide 2-[2-(3-méthoxy-phényl)-2,3-dihydro-1H-isoindol-5-ylamino]-5-nitro-benzoïque ;
acide 2-[2-(3-fluoro-phényl)-2,3-dihydro-1H-isoindol-5-ylamino]-benzoïque ;
acide 2-[2-(3,4-dichloro-phényl)-2,3-dihydro-1H-isoindol-5-ylamino]-5-fluoro-benzoïque ; et
acide 2- [2- (3,4-dichloro-phényl) -2,3-dihydro-1H-isoindol-5-ylamino]-nicotinique.

**12.** Les composés selon la revendication 1 :

acide 2-[2-(3,4-dichloro-phényl)-2,3-dihydro-1H-isoindol-5-ylamino]-5-méthoxy-benzoïque ;
acide 2-[2-(3,4-dichloro-phényl)-2,3-dihydro-1H-isoindol-5-ylamino]-3-nitro-benzoïque ;
acide 3-nitro-2-{2-[(4aS,8aR)-4-(octahydro-isoquinolin-2-yl)-phényl]-2,3-dihydro-1H-isoindol-5-ylamino}-benzoïque ;
acide 2-{2-[(4aS,8aR)-4-(octahydro-isoquinolin-2-yl)-phényl]-2,3-dihydro-1H-isoindol-5-ylamino}-benzoïque ;
acide 4-[2-(3,4-dichloro-phényl)-2,3-dihydro-1H-isoindol-5-ylamino]-nicotinique ;
acide 2-[2-(4-dibutylamino-phényl)-2,3-dihydro-1H-isoindol-5-ylamino]-benzoïque ;
acide 2-[2-(3-dibutylamino-phényl)-2,3-dihydro-1H-isoindol-5-ylamino]-benzoïque ;
acide 2-[2-(3-bromo-phényl)-2,3-dihydro-1H-isoindol-5-yl amino]-benzoïque ;
acide 2-[2-(2-chloro-phényl)-2,3-dihydro-1H-isoindol-5-ylamino]-benzoïque ;
acide 5-dibutylamino-2-[2-(3,4-dichloro-phényl)-2,3-dihy dro-1H-isoindol-5-ylamino]-benzoïque ;
acide 2-[2-(3,4-dichloro-phényl)-2,3-dihydro-1H-isoindol-5-ylamino]-5-méthoxy-benzoïque ;
acide 4-[2-(3,4-dichloro-phényl)-2,3-dihydro-1H-isoindol-5-ylamino]-isophthalique ;
acide 2- (2-biphényl-4-yl-2,3-dihydro-1H-isoindol-5-ylamino)-benzoïque ;
acide 2-[2-(3,4-diméthoxy-phényl)-2,3-dihydro-1H-isoindol-5-ylamino]-benzoïque ;
acide 2-[2-(3,4-dihydroxy-phényl)-2,3-dihydro-1H-isoindol-5-ylamino]-benzoique ;
acide 2-[2-(3,4-difluoro-phényl)-2,3-dihydro-1H-isoindol-5-ylamino]-benzoïque ;
acide 2-[2-(3-fluoro-4-méthyl-phényl)-2,3-dihydro-1H-isoindol-5-ylamino]-benzoïque ;
acide 2-[2-(3,4,5-trihydroxy-phényl)-2,3-dihydro-1H-isoindol-5-ylamino]-benzoïque ;
acide 2- [2- (4-méthyl-3-trifluorométhyl-phényl) -2,3-dihydro-1H-isoindol-5-ylamino] -benzoique ;
acide 2-[2-(3,5-dichloro-phényl)-2,3-dihydro-1H-isoindol-5-ylamino]-benzoïque ;
acide 2-[2-(2,4-dichloro-phényl)-2,3-dihydro-1H-isoindol-5-ylamino]-benzoïque ;
acide 2-[2-(4-fluoro-3-trifluorométhyl-phényl)-2,3-dihydro-1H-isoindol-5-ylamino]-benzoïque ;
2-[2-(3,4,5-triméthoxy-phényl)-2,3-dihydro-1H-isoindol-5-ylamino]-benzonitrile ; ou
acide 2-[2-(3-méthoxy-phényl)-2,3-dihydro-1H-isoindol-5-ylamino]-benzoïque.

**13.** Composition pharmaceutique comprenant un composé selon la revendication 1, conjointement avec un support, un diluant, ou un excipient pharmaceutiquement acceptable pour celui-ci.

**14.** Utilisation d'un composé selon les revendications 1 à 12, pour la fabrication de produits pharmaceutiques destinés au traitement de la maladie d'Alzheimer ou à l'inhibition de l'agrégation de protéines amyloïdes pour former des dépôt amyloïdes.

**15.** Utilisation d'un composé marqué selon les revendications 1 à 12 pour la fabrication de produits diagnostiques destinés à l'imagerie et à la détection de dépôts amyloïdes.